(19) **Europäisches Patentamt / European Patent Office / Office européen des brevets**

(11) **EP 4 218 808 A2**

(12) **EUROPEAN PATENT APPLICATION**

(43) Date of publication:
02.08.2023 Bulletin 2023/31

(21) Application number: 23153466.0

(22) Date of filing: 12.03.2013

(51) International Patent Classification (IPC):
**A61K 39/39** (2006.01)

(52) Cooperative Patent Classification (CPC):
**A61K 39/39;** A61K 2039/55555; A61K 2039/55577

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.03.2012 US 201261609783 P**

(62) Document number(s) of the earlier application(s) in accordance with Art. 76 EPC:
**13761420.2 / 2 825 196**

(71) Applicant: **Advanced BioAdjuvants, LLC Omaha, NE 68114 (US)**

(72) Inventors:
• **GERBER, Jay, D.**
  **Lincoln, 68502 (US)**

• **CARROW, Emily**
  **Higganum, 06441 (US)**

(74) Representative: **Mewburn Ellis LLP**
  **Aurora Building**
  **Counterslip**
  **Bristol BS1 6BX (GB)**

Remarks:
•This application was filed on 26-01-2023 as a divisional application to the application mentioned under INID code 62.
•Claims filed after the date of filing /receipt of the divisional application (Rule 68(4) EPC).

(54) **ADJUVANT AND VACCINE COMPOSITIONS**

(57) Methods are provided for preparing and delivering an adjuvant for vaccines including lecithin, polymer and one or more additives. The polymer is preferably polyacrylic acid-based. The additive is preferably one or more of a glycoside and a sterol. The method of preparation includes hydrating lecithin and a polymer in saline or water and mixing the lecithin and polymer to form the adjuvant. Additives can be included prior to or after hydration of the lecithin and polymer.

**Mean HAI Titer: DOWE8009-05-0B**

FIG. 1

## Description

This application is being filed on 12 March 2013, as a PCT International patent application, and claims priority to U.S. Provisional Patent Application No. 61/609,783, filed March 12, 2012, the disclosure of which is hereby incorporated by reference herein in its entirety.

FIELD

Provided herein are compositions and methods for preparing and delivering vaccine to a patient or animal in need thereof and in particular, to compositions and methods for preparing novel adjuvant compositions and delivering vaccines that include these novel adjuvant compositions to a patient or animal in need thereof.

BACKGROUND

Mucosal delivery of vaccines has been underutilized because of the problems associated with effectively delivering the vaccine antigens to the mucosal surface and to the underlying mucosal lymphoid tissue. Since mucosal surfaces are the port of entry of the majority of the infectious agents (Sabin, A. B., Vaccination at the portal of entry of infectious agents. Dev Biol Stand 33:3-9, 1976) it is important to the health of an animal to have developed a strong protective antibody and cell-mediated immune response at the portal of entry. This is best done with an adjuvant and delivery system that targets vaccine antigens to either the mucous membranes of the oral cavity, gut, nose, rectum, or vagina. Because this is not commonly done with an injectable vaccine, it would be advantageous to have a vaccine adjuvant delivery composition that would adsorb the vaccine onto the mucosal surface, and then, following absorption, be brought in contact with mucosal-associated lymphoid tissue.

For example, oral administration of a vaccine against a gut pathogen may engender a stronger immune response against such pathogens by eliciting the production of secretory immunoglobulin A antibodies at the mucosal site. This happens when the vaccine is presented to the gut-associated lymphoid tissue (O'Hagen, D, Oral Delivery of Vaccines: Formulation and Clinical Pharmacokinetic Considerations 1992, Clin. Pharmacokinet. 22 (1): 1-10). Likewise, administration of vaccine against an upper respiratory pathogen may be most effective if delivered to the mucosal-associated lymphoid tissue in the oral cavity or nasal passages. Interestingly, administration of antigens induces a mucosal immune response not only at the site of antigen application, for example the oral mucosa, but also at other mucosal sites such as the nasal mucosal (Mestecky, JI, The Common Mucosal Immune System and Current Strategies for Induction of Immune Responses in External Secretions. J Clin Immunol. 7 (4): 265-76).

Vaccinating large numbers of animals, such as cattle, swine and poultry, is extremely labor intensive and expensive. Each individual animal has to be handled at the time of vaccination in order to inject the animal with the vaccine. Most often the vaccine must be administered to the animal at least twice, and sometimes three or more times. It would be advantageous in terms of time and expense if the vaccine could be administered, simultaneously, with feed or water to a large number of animals.

Another advantage of targeting the vaccine to mucosal surfaces is that the vaccine can stimulate a protective immune response in the presence of circulating antibody that interferes with parenterally injected vaccines (Periwal, SB, et. al., Orally administered microencapsulated reovirus can bypass suckled, neutralizing maternal antibody that inhibits active immunization of neonates. J Virol 1997 (Apr 71(4): 2844-50)).

Adjuvant systems to enhance an animal's immune response to a vaccine antigen are well known. Likewise, systems for the delivery of vaccine and drugs to mucosal surfaces are known. Different methods have been described to protect the vaccine antigen and drugs from degradation by stomach acid and digestive enzymes and to adsorb the antigen to the mucosal surface. Often these adjuvants and delivery systems include mixing the antigen with one or more components.

Exemplary adjuvants include the following:

U.S. Pat. No. 4,917,892, Speaker et al, issued Apr. 17, 1990, describes a topical delivery system comprising a viscous carrier containing a dissolved or dispersed active agent and active agent microencapsulated within a semi permeable anisotropic salt film which is the emulsion reaction product of a) a partially lipophilic, partially hydrophilic, polyfunctional Lewis acid or salt thereof in aqueous medium, such as carboxymethylcellulose, an alkali metal salt of polyacrylic acid or cross linked polyacrylic acid/polyoxyethylene, with b) a Lewis base or salt thereof in a water-immiscible, slightly polar organic solvent for the base, such as benzalkonium chloride, and piperidine. U.S. Pat. No. 5,132,117, Speaker et al., issued Jul. 21, 1992, discloses a microcapsule with an aqueous core, capsular, ionic stabilized anisotropic Lewis salt membrane formed from the interfacial reaction product of an emulsion of an aqueous solution of a water-soluble, hydrophilic polymeric Lewis acid or salt thereof with a non-aqueous solution of a lipophilic Lewis base or salt thereof. The Lewis base may be stearylamine, piperidine, or benzalkonium chloride and the Lewis acid may be carboxymethylcellulose, polyacrylic acid, or polyacrylic acid/polyoxyethylene copolymer, for example.

U.S. Pat. No. 4,740,365, Yukimatsu et al., issued Apr. 26, 1988 describes a sustained-release preparation applicable to mucous membranes in the oral cavity. The preparation consists of an active ingredient in a mixture of a polymer component (A) comprising one or more polymers selected from polyvinylpyrrolidone, polyvinyl alcohol, polyethylene glycol, alginic acid or a salt thereof, and an alternating copolymer of maleic anhydride and methyl vinyl ether and a polymer component (B) comprising one or more polymers selected from polyacrylic acid and a salt thereof. Polymer component (A) and (B) are in a ratio of 95:5 to 5:95 by weight. The preparation is layered with the active ingredient and may have optional conventional carriers and additives.

U.S. Pat. No. 5,451,411, Gombotz et al., issued Sep. 19, 1995, describes a delivery system for a cationic therapeutic agent whereupon alginate has been cross-linked in the presence of the therapeutic agent and polyacrylic acid to obtain a sustained release composition for oral delivery.

U.S. Pat. No. 5,352,448, Bowersock et al., issued Oct. 4, 1994, describes an oral vaccine formulation comprising an enzymatically degradable antigen in a hydrogel matrix for stimulation of an immune response in gut-associated lymphoid tissues. The hydrogel pellets are preferably synthesized by polymerizing methacrylic acid, in the presence of methylene bis-acrylamide and ammonium persulfate and sodium bisulfite.

U.S. Pat. No. 5,674,495, Bowersock et al., issued Oct. 7, 1997, describes a vaccine composition for oral administration comprising an alginate gel in the form of discrete particles. The alginate gel may contain a polymer coating such a poly-l-lysine to enhance stability and to add a positive charge to the surface.

U.S. Pat. No. 4,944,942, Brown et al., issued Jul. 31, 1990, describes an intranasal vaccine for horses, which may comprise polyacrylic acid cross linked polyallyl sucrose combined with polyoxyethylene sorbitan mono-oleate and sorbitan monolaurate, preferably at 7.5 to 15 volume percent based on the total volume of the formulation, as an adjuvant.

U.S. Pat. No. 5,500,161, Andrianov et al., issued Mar. 19, 1996, describes a method for the preparation of microparticles, and the product thereof, that includes dispersing a substantially water insoluble non-ionic or ionic polymer in a aqueous solution in which the substance to be delivered is also dissolved, dispersed or suspended, and then coagulating the polymer together with the substance by impact forces to form a microparticle. Alternatively, the microparticle is formed by coagulation of an aqueous polymeric dispersion through the use of electrolytes, pH changes, organic solvents in low concentrations, or temperature changes to form polymer matrices encapsulating biological materials.

U.S. Pat. No. 6,015,576, See et al., issued Jan. 18, 2000, describes a method that comprises orally administering lyophilized multilamellar liposomes containing the antigen wherein the liposome preparation is contained in a pill form or within an enterically coated capsule. Such an enteric coating may be composed of acrylic polymers and copolymers.

U.S. Pat. No. 5,811,128, Tice et al., issued Sep. 22, 1998, describes a method, and compositions for delivering a bioactive agent to an animal entailing the steps of encapsulating effective amounts of the agent in a biocompatible excipient to form microcapsules having a size less than approximately ten micrometers and administering effective amounts of the microcapsules to the animal. A pulsatile response is obtained, as well as mucosal and systemic immunity. The biocompatible excipient is selected from the group consisting of poly (DL-lactide-co-glycolide), poly (lactide), poly (glycolide), copolyoxalates, polycaprolactone, polyorthoesters and poly (beta-hydroxybutyric acid), polyanhydrides and mixtures thereof.

U.S. Pat. No. 5,565,209, Rijke, issued Oct. 15, 1996, describes oil-free vaccines comprising polyoxypropylene-polyoxyethylene polyols and an acrylic acid polymer as adjuvant constituents for injectable vaccines.

U.S. Pat. No. 5,084,269, Kullenberg, issued Jan. 28, 1992, describes an adjuvant, comprised of lecithin in combination with a carrier which may be selected from the group consisting of non-edible oil such as mineral oil and edible triglyceride oils such as soybean oil, for an injectable vaccine.

U.S. Pat. No. 5,026,543, Rijke, issued Jun. 25, 1991, discloses oil-free vaccines which contain polyoxypropylene-polyoxyethylene polyols as well as an acrylic acid polymer as adjuvanting constituents.

U.S. Pat. No. 5,451,411, Gombotz et al, issued Sep. 19, 1995, discloses alginate beads as a site specific oral delivery system for cationic therapeutic agents designed to target the agents to the luminal side of the small intestine. Enhanced bioactivity of therapeutic agents released from the alginate is attributed to the ability of polyacrylic acid to shield the agents from interaction with lower molecular weight fragments of acid treated alginate.

U.S. Pat. No. 5,567,433, Collins, issued Oct. 22, 1996, discloses a method of producing liposomes useful for encapsulating and delivering a wide variety of biologically active materials. The method involves the formation of a liposome dispersion in the absence of an organic solvent or detergent, one or several cycles of freezing and thawing, and dehydration to form a lipid powder. The powder is hydrated in the presence of a biologically active material to encapsulate it in the liposomes.

U.S. Pat. No. 5,091,188, Haynes, issued Feb. 25, 1992, discloses water-insoluble drugs rendered injectable by formulation as aqueous suspensions of phospholipid-coated microcrystals.

SUMMARY

The present invention concerns an adjuvant composition that includes lecithin and a polymer that is preferably an acrylic polymer or copolymer. An exemplary acrylic polymer is a polyacrylic acid polymer. Any lecithin is contemplated herein, including individual phospholipid components of lecithin or any combination thereof. In some embodiments, the present invention also concerns lecithin and polymer adjuvant compositions that include one or more additives that facilitate an immune response, including glycosides, sterols, ISCOMS, muramyl dipeptide and analogues, pluronic polyols, trehalose dimycolate, amine containing compounds, cytokines, calcium and lipopolysaccharide derivatives. Exemplary additives are glycosides and sterols, where the glycoside can be Quil A and the sterol can be cholesterol.

The present invention also includes an adjuvant composition that consists of only a lecithin and polymer, and does not include additional lipid components. Typical polymers are acrylic polymer or copolymer. In one particular embodiment the adjuvant consists of lecithin and polyacrylic acid polymer.

The present invention also includes an adjuvant composition that consists of a lecithin and polymer adjuvant composition in combination with one or more glycosides and/or one or more sterols. In some embodiments the adjuvant composition consists of lecithin, polymer, a glycoside and a sterol, where the glycoside can be a saponin or any fraction thereof and the sterol can be, for example, cholesterol. In some embodiments the polymer is an acrylic polymer or copolymer, for example, polyacrylic acid polymer.

In general, the lecithin and polymer adjuvants herein form a matrix or net-like structure which is effective in trapping or encapsulating vaccine antigen. In some cases, the lecithin and polymer adjuvant combination form an "oil-free" net-like structure, being composed predominately (and in some cases entirely) by phospholipids and acrylic polymer. In other cases, the lecithin and polymer adjuvant includes additives directed toward further facilitating the adjuvant's capacity to elicit an immune response.

The strong mucoadhesive and adsorptive properties of the polymer and lecithin combination enhances the adsorption of vaccine antigen onto mucosal surfaces. Further, the lecithin composition enhances absorption (Swenson, ES and WJ Curatolo, ©Means to Enhance Penetration (2) Intestinal permeability enhancement for proteins, peptides and other polar drugs: mechanisms and potential toxicity. Advanced Drug Delivery Reviews. 1992. 8:39-92) that helps bring the antigen in contact with the underlying lymphoid tissue. Embodiments herein provide a significant improvement over conventional vaccines for delivery of an antigen to a mucosal surface, particularly where the adjuvant does not include the significant proportion or ratio of polymer, as shown in the inventive embodiments herein.

The adjuvant compositions of this invention make it possible to vaccinate via a mucosal surface, such as oral cavity, gut, nasal, rectal, or vaginal surfaces. The vaccine may be administered by pill or tablet form, a paste form or in fluid form using a dropper or needleless syringe. This adjuvant composition allows a method of vaccination via food and/or water. In addition, the adjuvant compositions herein facilitate robust mucosal immunity, an advancement over conventional administration techniques for a number of antigens.

In an alternative embodiment the composition can be used traditionally as an injectable.

Thus, there is provided a method for preparing an adjuvant composition comprising: hydrating lecithin and a polymer in saline or water; and mixing the lecithin and polymer to form an adjuvant.

In some embodiments, the lecithin and the polymer can be mixed by placing the lecithin and the polymer in a blender.

Advantageously, the lecithin and the polymer can be mixed in the presence of surfactants. In some instances the lecithin and polymer are mixed in the presence of other additives, for example: a glycoside and/or sterol.

In some embodiments, the method further includes the step of microwaving or autoclaving the adjuvant. In some embodiments, the method further includes the step of not filtering the adjuvant.

In one embodiment, from about 0.001-10% by weight dry lecithin and from about 0.001-10% by weight polymer are hydrated. In some implementations the polymer is also dry and the lecithin and polymer are mixed dry prior to hydration. In this implementation, the method further includes the step of adding an antigen. Advantageously, the antigen is added during the hydration step. In another advantageous embodiment, the antigen is added to the adjuvant.

The lecithin and the polymer can be mixed in the presence of an oil.

Adjuvants of the invention can be mixed by placing the lecithin and the polymer in a microfluidizer.

Alternative implementations include adding a calcium based compound to the adjuvants described herein where a DNA based antigen is implemented in the vaccine.

Further features and advantages of the present invention will be set forth in, or apparent from, the detailed description which follows.

BRIEF DESCRIPTION OF THE FIGURES

Figure 1 is a bar graph showing mean HAI titer for various antigen concentrations and adjuvants.

Figure 2A and Figure 2B are micrographs at 30,000X magnification of an embodiment adjuvant composition of the present invention (2A) and a competitor's adjuvant composition (2B).

Figure 3 shows data from HAI GMT serum titers for various adjuvant embodiments of the present invention.

Figure 4 shows the results of Adenovirus vector-based FMD vaccine alone or in combination with adjuvant embodiments described herein.

Figure 5, Figure 6 and Figure 7 are cell viability bar graphs having a starting cell density of $1 \times 10^6$/ml, percent reduction of Alamar Blue at 42 hrs (5), 50 hrs (6) and 68 hrs (7) from treatment.

Figure 8, Figure 9 and Figure 10 are cell viability bar graphs having a starting cell density of $10 \times 10^6$/ml, percent reduction of Alamar Blue at 42 hrs (8), 50 hrs (9) and 68 hrs (10) from treatment.

Figure 11 and Figure 12 show Log 10 $TCID_{50}$ of adjuvant options with Ad5bIFNalpha-adjuvant mixtures after incubation (11) and Log 10 $TCID_{50}$ of adjuvant options with Ad501-adjuvant mixtures after incubation) (12).

Figure 13A and Figure 13B are graphs showing group average of IL-4 expression at 24 and 48 hours normalized to ARBP.

Figure 14 is a graph showing average IL-4 expression at 48 hours normalized to HPRT.

Figure 15A and 15B are graphs showing a group average of IFN-□ expression at 25 and 48 hours normalized to ARBP or HPRT.

Figure 16A and 16B are graphs showing a group average of TNF-alpha expression at 24 and 48 hours normalized to HPRT.

Figure 17 is a graph showing average IL-4 expression at 24 and 48 hours treatment with LAP + OVA or LAP/QAC + OVA normalized to HPRT.

DETAILED DESCRIPTION

The present invention provides a vaccine adjuvant which, when admixed with an antigen or hapten and administered into a human or animal, will induce a more intense immune response to the antigen than when the antigen is administered alone. The present invention also provides vaccines comprising an antigen or group of antigens and a novel adjuvant herein described which comprises a combination of lecithin and a polymer. As will appear, the present invention also specifically provides methods of making and using the foregoing adjuvants and vaccines.

Such adjuvants offer the advantage of allowing application of a vaccine directly to a mucosal surface. In doing so, the vaccine stimulates a protective immune response which helps prevent interference from circulating maternal antibodies that may be present in a newborn or infant, for example. Direct administration of a vaccine herein to a mucosal surface, i.e., mucosal vaccination, provides mucosal immunity and systemic immunity, an advantage over most systemic only based vaccines. Unlike other vaccines developed for mucosal vaccination, embodiments of the present invention provide unexpected improvement for immunogenic response by improving the vaccine's contact time on the mucosal surface.

"Antigen" is herein defined as a compound which, when introduced into an animal or a human, will result in the formation of antibodies and cell-mediated immunity.

"Adjuvant" is herein defined as a compound or compounds that, when used in combination with specific vaccine antigens in formulations, augment or otherwise alter or modify the resultant immune responses.

"Vaccine" is herein defined as a composition of antigenic moieties, usually consisting of modified-live (attenuated) or inactivated infectious agents, or some part of the infectious agents, that is administered, most often with an adjuvant, into the body to produce active immunity.

The antigen can be any desired antigen falling within the definition set forth above. Antigens are commercially available or one of skill in the art is capable of producing them. The antigenic moiety making up the vaccine can be either a modified-live or killed microorganism, or a natural product purified from a microorganism or other cell including, but not limited to, tumor cells, a synthetic product, a genetically engineered protein, peptide, polysaccharide or similar product, or an allergen. The antigenic moiety can also be a subunit of a protein, peptide, polysaccharide or similar product. The antigen may also be the genetic antigens, i.e., the DNA or RNA that engenders an immune response. Representative of the antigens that can be used according to the present invention include, but are not limited to, natural, recombinant or synthetic products derived from viruses, bacteria, fungi, parasites and other infectious agents in addition to autoimmune diseases, hormones, or tumor antigens which might be used in prophylactic or therapeutic vaccines and allergens. The viral or bacterial products can be components which the organism produced by enzymatic cleavage or can be components of the organism that were produced by recombinant DNA techniques that are well known to those of ordinary skill in the art. Because of the nature of the invention and its mode of delivery it is very conceivable that the invention would also function as a delivery system for drugs, such as hormones, antibiotics and antivirals.

The lecithin can be any lecithin or, for instance, lecithin lipoidal material, such as phospholipids, lysophospholipids, glycolipids and neutral lipids that comprise the Typical composition of lecithin. Lecithins are molecules that, when completely hydrolyzed, yield two molecules of fatty acid, and one molecule each of glycerol, phosphoric acid, and a basic nitrogenous compound, such as choline. The fatty acids obtained from lecithins on hydrolysis are usually, but not limited to, oleic, palmitic, and stearic acids. The phosphoric acid may be attached to the glycerol in either an α- or the β-position, forming α-glycerophosphoric acid or β-glycerophosphoric acid, respectively, and producing the corresponding series of lecithins which are known as α- and β-lecithins.

Commercial lecithin is obtained by extraction processes from egg yolk, brain tissue, or soybeans. Ovolecithin (vitelin) from eggs and vegilecithin from soybeans, as well as purified lecithin from calfs brains have been used as emulsifiers, antioxidants, and stabilizers in foods and pharmaceutical preparations. Commercial lecithin may be obtained from a variety of sources. One of ordinary skill in the art would be able to determine an appropriate lecithin for a desired application.

The polymer is preferably an acrylic polymer, which is any polymer or copolymer that contains an acrylic moiety. Examples of suitable acrylic polymers include, but are not limited to polyacrylic acid, methacrylic acid, methacrylate, acrylamide, acrylate, acryinitrile, and alkyl-esters of poly acrylic acid. Examples of acrylic copolymers are poly (acrylamide-co butyl, methacrylate), acrylic-methacrylic acid, acrylic-acrylamide and poly (methacrylate). Commercial polymers may be obtained from a variety of sources.

In some embodiments, acrylic polymers may benefit from the inclusion of a cross linker, such as a polyalkenyl polyether, an alkyl sucrose, or an allyl ether of penta-erythirtol, for example, which is effective in binding the polymers. An exemplary acrylic polymer for use in this invention is polyacrylic acid with or without a polyalkenyl polyether cross linker. One of ordinary skill in the art would be able to determine an appropriate acrylic polymer for a desired application. Likewise, one of ordinary skill in the art would be able to determine an appropriate cross linker for a given acrylic polymer.

Examples of non-acrylic polymers that are suitable for use herein are polyvinyl acetate phthalate, cellulose acetate phthalate, methylcellulose, polyethylene glycol, polyvinyl alcohol, and polyoxyethylene.

The method of manufacturing the adjuvant of this invention first involves hydrating the lecithin and polymer by suspending from about 0.0001-10% by weight/volume dry lecithin and from about 0.0001-10% by weight polymer in saline or water. In some cases the polymer is also dry prior to suspension in saline or water. The preferred concentrations of lecithin and polymer are 0.001-1.0% each by weight/volume. The two components may be mixed together using conventional methods, such as, for example, a Waring Blender, emulsification equipment or a microfluidizer. Surfactants (emulsifiers) may be added to aid in the mixing or emulsification of the lecithin and polymer. Suitable synthetic detergents are well known to those of ordinary skill in the art. Examples of appropriate surfactants include polyoxyethylene sorbitan monooleate, sorbitan monolaurate, sodium stearate, non-ionic ether-linked surfactants such as Laureth®4 and Laureth®23, alkyl sulfate surfactants, alkyl alkoxylated sulfate surfactants, alkylbenzenesulphonates, alkanesulphonates, olefinsulphonates, sulphonated polycarboxylic acids, alkyl glycerol sulfonates, fatty acyl glycerol sulfonates, fatty oleyl glycerol sulfates, alkyl phenol ethylene oxide ether sulfates, paraffin sulfonates, alkyl phosphates, isothionates such as the acyl isothionates, N-acyl taurates, fatty acid amides of methyl tauride, alkyl succinamates and sulfosuccinates, mono- and diesters of sulfosuccinate, N-acyl sarcosinates, sulfates of alkylpolysaccharides, branched primary alkyl sulfates, alkyl polyethoxy carboxylates, and fatty acids esterified with isethionic acid and neutralized with sodium hydroxide. Further examples are given in Surface Active Agents and Detergents (Vol. I and II by Schwartz, Perry and Berch), the disclosure of which is expressly incorporated herein by reference. Suitable nonionic detergent surfactants are generally disclosed in U.S. Pat. No. 3,929,678, Laughlin et al., issued Dec. 30, 1975, at column 13, line 14 through column 16, line 6, incorporated herein by reference. If included, the emulsifier should be added in a concentration ranging from about 0.001-0.05% by volume of the mixture.

Lecithin and polymer embodiments herein may also be used in combination with other additives such as, but not limited to, glycosides such as saponins, fractions of saponins, or synthesized components of saponins, sterols, ISCOMS, muramyl dipeptide and analogues, pluronic polyols, trehalose dimycolate, amine containing compounds, cytokines, calcium and lipopolysaccharide derivatives. The addition of another additive may aid in the stimulation of an immune response and in particular a mucosal immune response. If included, additional additives may be present in a concentration of up to about 10% by weight of the composition, for example, less than about 1% by weight of the composition. In most cases, additives of the invention are included in adjuvant embodiments herein in an amount to cause an induction of an immune response. In some instances, additives herein provide additional stimulation of TH1 (T helper cell 1), an important mediator in mucosal immunity.

Typical additives for inclusion with lecithin/polymer adjuvants described herein include one or more of a glycoside and/or a sterol. In some embodiments, the glycosides are saponins, fractions of saponins or synthesized components of saponins. Exemplary saponins can be derived from plant sources including, but not limited to *Quillaja Saponaria* Molina, *Polygala senega,* and *Astragalus* species. In one instance the saponin is a triterpensaponin, such as Quil A (a saponin preparation isolated from the South American tree *Quillaja Saponaria* Molina). Purified fractions of Quil A include QS7 and QS21 (also known as QA7 and QA21). Nonetheless, any saponins are contemplated as useful herein. Preferred sterols are cholesterol, lanosterol, lumisterol, stigmasterol and sitosterol.

In some instances, Quil A is combined with cholesterol and added to adjuvant embodiments described herein.

This particular additive combination provides an unexpected enhancement in immune response over similarly prepared lecithin and polymer adjuvants described herein. Note that QS7 and/or QS21 can be substituted and/or added to the Quil A.

Typical additives for inclusion with lecithin/polymer adjuvants described herein also include calcium compounds, for example, calcium phosphate, as described in US Patent Application S/N 12/125,577, incorporated herein by reference for all purposes. Calcium additives are most appropriate for DNA virus based antigens, although it is envisioned that other antigen types can be used. In some instances, calcium compounds can be combined with Quil A and/or cholesterol, and included in a lecithin/polymer based adjuvant.

The invention may also include one or more probiotics. Probiotics are bacteria or microorganisms that are beneficial to the health of the individual or animal. Examples of commonly used probiotics include, but are not limited to, various beneficial strains of Lactobacillus, Bifidobacterium, Streptococcus, etc. If present, each of the organisms should be administered in a concentration ranging from about $10^3$ to $10^8$ CFU each (per vaccination).

In addition to all of the above, as is well understood by those skilled in the art, other minors can be employed to make the composition more pharmaceutically and/or cosmetically elegant. For example, dyes can be added at very minor levels as can diluents such as alcohol, buffers, stabilizers, wetting agents, dissolving agents, colors, etc. With the exception of diluents such as alcohols which are used at higher levels, the levels of these minors are generally not more than 0.001% to 1.0% by weight.

If desired, the adjuvant components may be sterilized by autoclaving prior to the hydration step. It has also been found that autoclaving and/or microwaving the components may improve their suspending ability. The vaccine antigen may be added after formation of the adjuvant, or at the time of hydration of the adjuvant components. If in tablet form, the antigen may be mixed with dry components of the adjuvant invention along with other excipients necessary for tablet formation. Examples of appropriate types of vaccine antigens include killed or attenuated bacterial, viral, parasitic, or subunits of these organisms, or genomic vaccine antigens, for example, DNA.

Applicant believes that the capacity to be autoclaved/microwaved provides a significant benefit over other conventional adjuvants which require filtration. Initially, structural analysis of embodiments herein before and after autoclaving illustrated that the structure of the autoclaved composition(s) was not significantly affected. However, the more costly filtration method for sterilizing an adjuvant removes and/or modifies structural aspects of the composition. As such, the embodiments herein are relatively less costly and avoid structural alterations found in other adjuvant materials that require filtration. This is an unexpected finding of the present formulations.

The relative concentration of the components, including the antigen, may be determined by testing the formulations in animals starting with a low dose of the formulation and then increasing the dosage while monitoring the immune response. The following considerations should be made when determining an optimal dose, e.g., breed, age, size and the presence or absence of interfering maternal antibodies.

A concentration of an attenuated viral vaccine will comprise about $10^3$ to $10^9$ $TCID_{50}$ per animal. In some embodiments, the amount will be from about $10^4$ to $10^7$ $TCID_{50}$ per animal. The concentration of killed antigen or subunit antigen may range from nanogram to milligram quantities of antigen with about 1 microgram to 1 milligram preferred.

When the acrylic polymer and lecithin are combined, a matrix, or net-like structure is formed. In some instances the net-like structure is "oil free," i.e., not having additional oils or lipids added to the adjuvant. The ratio of lecithin to polymer include ratios between 1:1000 and 1000:1. In some embodiments, the ratios of lecithin to polymer include ratios between 1:10 and 10 to 1. The relative proportions of lecithin and acrylic polymer may be found to be important to the efficiency of delivery of different antigens, i.e., bacterial, viral, parasitic or sub-units of these organisms. The optimum ratio may be determined by the conventional means of testing the different ratios of lecithin to polymer with the desired antigen in animals.

The adjuvant composition can be used for the delivery of vaccine antigens such as whole killed or attenuated virus, bacteria, or parasite vaccine antigens or sub-unit(s) of such organisms to mucosal surfaces, such as oral cavity, gut, nasal, vaginal and rectal surfaces. Electron microscopic evaluation shows that there exists a physical and/or chemical affinity between lecithin and polymer. This affinity or association appears as a matrix, or net-like structure. Without intending to be bound by any particular theory, it is believed that a structure such as this can function as a means of physically trapping or encapsulating vaccine antigen. Such binding of antigen is further enhanced by the electrical charge and the hydrophilic and hydrophobic properties of lecithin and the acrylic polymers of this invention. To facilitate this, a polymer of different electrical charge may be selected depending on the anionic or cationic properties of the antigen. Likewise a polymer and lecithin of different hydrophobicity may be selected depending on the lipophilic or hydrophilic properties of the antigen.

If necessary or desired, the antigen can be coupled to the lecithin-acrylic polymer matrix with a cross-linker such as glutaraldehyde in a concentration of from about 1 to 50 mM, for example, about 15 mM. Further, the antigen can be coupled using water-soluble carbodiimide in a concentration ranging from about 0.05-0.5 M, for example, about 0.1 M, or a coupling method using a heterofunctional reagent such as N-hydroxysuccinimidyl 3-(2-pyridyldithio) propionate (SPDP) in a concentration ranging from about 0.1-1.0 mM, and preferably about 0.2 mM. Other appropriate coupling agents include mixed anhydride and bisdiazotized benzidene. The cross-linker is used to improve the binding affinities of the components of the adjuvant composition, for example, where the components are not electrically attracted to each other.

The strong mucoadhesive and adsorptive properties of the polymer, e.g., acrylic acid and lecithin combination also make it an excellent mechanism to aid in the adsorption of vaccine antigen onto mucosal surfaces. The adjuvant delivery system's absorption enhancement properties help bring the vaccine antigen in contact with mucosal associated lymphoid tissue. Thus, an immune response is engendered that will aid in the protection of an animal from infections and/or disease process. A robust mucosal immune response is critical since most infectious disease-causing organisms gain entry to the animal at mucosal surfaces. The invention can also be used as an adjuvant for injectable vaccines and provides improvement for facilitating an immune response over other conventional injectable adjuvant materials.

The vaccine comprising the adjuvant is delivered to a mucosal surface by direct application, ingestion through the oral cavity, insertion, injection, and through other conventional means known in the art. Alternatively, the adjuvant may also be administered as a conventional injectable, which is typically either a liquid solutions or suspension. When administered in a food or beverage carrier, the adjuvant/vaccine composition of this invention is generally included in the carrier composition in a concentration ranging from about 0.0001-10% by weight/volume (w/v) in case of a beverage carrier and weight/weight (w/w) in case of a food carrier, for example, about 0.01-1.0% w/v or w/w respectively. When administered in an injectable, the adjuvant/vaccine composition should be present in a concentration ranging from about 0.02-2.0% by weight, for example, about 0.1-0.5% by weight.

The adjuvant/vaccine may also be administered in other conventional solid dosage forms, such as in tablets, capsules, granules, troches, and vaginal or rectal suppositories. If administered in a solid dosage form, the adjuvant/vaccine composition should constitute between 0.0001-10% by weight of the dosage form, for example, about 0.01-1.0% by weight.

In addition to the active compounds, the pharmaceutical compositions of this invention may contain suitable excipients and auxiliaries which facilitate processing of the active compounds into preparations which can be used pharmaceutically. Oral dosage forms encompass tablets, capsules, and granules. Preparations which can be administered rectally include suppositories. Other dosage forms include suitable solutions for administration parenterally or orally, and compositions which can be administered buccally or sublingually.

The pharmaceutical preparations of the present invention are manufactured in a manner which is itself well known in the art. For example the pharmaceutical preparations may be made by means of conventional mixing, granulating, disolving, lyophilizing processes. The processes to be used will depend ultimately on the physical properties of the active ingredient used.

Suitable excipients are, in particular, fillers such as sugars for example, lactose or sucrose mannitol or sorbitol, cellulose preparations and/or calcium phosphates, for example, tricalcium phosphate or calcium hydrogen phosphate, as well as binders such as starch, paste, using, for example, maize starch, wheat starch, rice starch, potato starch, gelatin, gum tragacanth, methyl cellulose, hydroxypropylmethylcellulose, sodium carboxymethylcellulose, and/or polyvinyl pyrrolidone. If desired, disintegrating agents may be added, such as the above-mentioned starches as well as carboxymethyl starch, cross-linked polyvinyl pyrrolidone, agar, or alginic acid or a salt thereof, such as sodium alginate. Auxiliaries are flow-regulating agents and lubricants, for example, such as silica, talc, stearic acid or salts thereof, such as magnesium stearate or calcium stearate and/or polyethylene glycol. Oral dosage forms may be provided with suitable coatings which, if desired, may be resistant to gastric juices.

For this purpose concentrated sugar solutions may be used, which may optionally contain gum arabic, talc, polyvinylpyrrolidone, polyethylene glycol and/or titanium dioxide, lacquer solutions and suitable organic solvents or solvent mixtures. In order to produce coatings resistant to gastric juices, solutions of suitable cellulose preparations such as acetylcellulose phthalate or hydroxypropylmethylcellulose phthalate, dyestuffs and pigments may be added to the tablet coatings, for example, for identification or in order to characterize different combination of compound doses.

Other pharmaceutical preparations which can be used orally include push-fit capsules made of gelatin, as well as soft, sealed capsules made of gelatin and a plasticizer such as glycerol or sorbitol. The push-fit capsules can contain the active compounds in the form of granules which may be mixed with fillers such as lactose, binders such as starches, and/or lubricants such as talc or magnesium stearate and, optionally, stabilizers. In soft capsules, the active compounds are preferably dissolved or suspended in suitable liquids, such as fatty oils, liquid paraffin, or liquid polyethylene glycols. In addition stabilizers may be added. Possible pharmaceutical preparations which can be used rectally include, for example, suppositories, which consist of a combination of the active compounds with the suppository base. Suitable suppository bases are, for example, natural or synthetic triglycerides, paraffin hydrocarbons, polyethylene glycols, or higher alkanols. In addition, it is also possible to use gelatin rectal capsules which consist of a combination of the active compounds with a base. Possible base material includes, for example, liquid triglycerides, polyethylene glycols, or paraffin hydrocarbons.

Suitable formulations for parenteral administration include aqueous solutions of active compounds in water-soluble or water-dispersible form. In addition, suspensions of the active compounds as appropriate oily injection suspensions may be administered. Suitable lipophilic solvents or vehicles include fatty oils for example, sesame oil, or synthetic fatty acid esters, for example, ethyl oleate or triglycerides. Aqueous injection suspensions may contain substances which increase the viscosity of the suspension, including for example, sodium carboxymethyl cellulose, sorbitol and/or dextran. Such compositions may also comprise adjuvants such as preserving, wetting, emulsifying, and dispensing agents. They may also be sterilized, for example, by filtration through a bacteria-retaining filter, or by incorporating sterilizing agents into the compositions. They can also be manufactured in the form of sterile solid compositions which can be dissolved or suspended in sterile water, saline, or other injectable medium prior to administration.

In addition to administration with conventional carriers, active ingredients may be administered by a variety of specialized delivery drug techniques which are known to those of skill in the art, such as portable infusion pumps.

The lecithin/polymer adjuvant serves multiple functions when it is delivered orally in food and water: 1) it protects the vaccine antigen from degradation by the stomach acid and digestive enzymes; 2) transports the antigen to the mucosal surfaces; 3) facilitates adsorption of the antigen onto the mucosal surfaces; 4) enhances absorption of the antigen; and 5) enhances the immune response to the antigen due to the adjuvant properties of the two components. In the case of delivery to nasal, oral cavity, vaginal and rectal mucosa, the lecithin/acrylic polymer complex functions as a system to deliver and adsorb the antigen to the mucosal surface. Once adsorbed onto the mucosal surface and absorbed, an immune response is engendered.

The combination of polymer and lecithin unexpectedly provides an improved vaccine delivery system for vaccine antigens. It is apparent that the invention is also an improved delivery system for drugs, such as hormones, antibiotics, probiotics and antivirals. The current invention provides a more simple and efficient method of incorporation of antigen into a delivery system with no, or minimal damage, to vaccine epitopes. The vaccine formulation can be done at low cost and can be easily commercialized as a feed or water additive or as an oral paste or tablet. It is to be understood that these formulations also would be effective in delivering antigen onto other mucosal surfaces, such as nasal, rectal and vaginal surfaces, and would be effective as an adjuvant for an injectable vaccine. In addition, the hydrophobic properties that aid in the adsorption of the adjuvant and vaccine antigen to mucosal surfaces also provides a means of applying to animal feeds, whether it be plant foliage or seeds, both which have a hydrophobic wax surface.

The combination of polymer, lecithin and additives (in particular Quil A and cholesterol) provides an unexpected enhancement of an adjuvant to induce an immune response. This benefit is present for multiple delivery routes, which includes when the adjuvant is delivered as an injectable.

The composition with which the current invention is concerned differs from the prior art in that it comprises a mixture of lecithin and, in some embodiments, an acrylic polymer or copolymer. The invention provides certain advantages over other vaccine delivery systems described in the prior art. It is not prepared under harsh conditions that adversely affect the substance such as the use of organic solvents. It does not require elevated temperatures to manufacture and does not require a stabilization step. The invention provides a simpler method of incorporation of antigen with minimal damage to vaccine epitopes. Using this simpler method of manufacturing results in low cost and ease of commercialization.

The following examples are intended to further illustrate the invention and its preferred embodiments. They are not intended to limit the invention in any manner.

EXAMPLE 1

Vaccine Plus Adjuvant Effectiveness

An experimental vaccine was made comprising bovine serum albumin Fraction 5 (BSA) as a non-living model antigen, lecithin, and an acrylic acid polymer. A second vaccine was made comprising only BSA.

The lecithin and acrylic polymer were suspended together in 150 milliliters (ml) phosphate buffered saline (PBS), each at a concentration of 4 milligrams (mg) per milliliter (ml). The components were first dispersed by stirring with a magnetic stir bar and then mixed further in a Waring Blender using an emulsification head. The mixture was then autoclaved to sterilize the adjuvant mixture. Bovine serum albumin was dissolved in PBS at a concentration of 2 mg/ml and filter sterilized. One part lecithin/acrylic polymer adjuvant was then combined with one part of BSA. Merthiolate (0.01%) was added as a preservative. The final concentration of the vaccine components was 2 mg/ml of the lecithin/acrylic polymer and 1 mg/ml of BSA.

CF-1 female mice, approximately 18 grams, from Charles River Laboratories (Willmington, Mich.), were injected subcutaneously in the groin area with 0.1 ml of vaccine (0.1 mg. of BSA/dose) on days 0 and 21. Mice were bled on day 45, 24 days after the second vaccination. Mice were bled by cutting the brachial artery following euthanasia by cervical dislocation.

Blood serum Immunoglobulin G (IgG) anti-BSA antibody titers were determined by an enzyme linked immunosorbant assay (ELISA). Results are shown in Table 1.

TABLE 1

| Results of Antibody Titers | | |
|---|---|---|
| Adjuvant Group | Number of Mice | Reciprocal of Geometric Mean Titer |
| None | 8 | 51,200 |
| Lecithin/Acrylic Polymer | 8 | 157,916 |

Results show that the adjuvant comprising a combination of lecithin and acrylic polymer does indeed enhance the immune response to an antigen.

EXAMPLE 2

Comparison of Individual Vaccine Adjuvants Administered Orally

Experimental vaccines, for delivery by the oral route, were prepared in PBS. The vaccines comprised the antigen, BSA Fraction 5, at a concentration of 400 micrograms ($\mu$g) per ml. Vaccine 1 contained no adjuvant only BSA. Vaccine 2 was comprised of BSA mixed with 3 mg/ml of lecithin. Vaccine 3 was comprised of BSA mixed with 3 mg/ml of the acrylic polymer. Vaccine 4 was comprised of BSA mixed with 3 mg/ml of lecithin and 3 mg/ml of acrylic polymer. Mixing was first done with a laboratory bench top magnetic stir bar and then in a Waring blender using an emulsification head. Lactobacillus culture was added to all vaccines just prior to vaccination. The final concentration of Lactobacillus was 0.01 $\mu$g/ml of vaccine. On days 0, 4, 29 and 33 the groups of CF-1 female mice from Charles-River Laboratories and weighing approximately 18 grams, were administered 0.5 ml of vaccine orally by feeding needle. On day 53, 20 days post fourth vaccination, mice were euthanized and bled by the brachial artery. End-point anti-BSA serum IgG antibody titers were determined by ELISA. A 1/100 starting dilution of serum was used due to non-specific background color development at dilutions less than 1/100. Results are recorded in Table 2:

TABLE 2

| Effect of Adjuvant Composition on the Anti-BSA Antibody Response | | |
|---|---|---|
| Adjuvant Composition | No. of Mice with Titer >/= 1/100 (%) | Reciprocal of Geometric Mean of Mice with Titers |
| None | 3/9 (33) | 158 |
| Lecithin | 4/6 (67) | 141 |
| Acrylic Polymer | 6/9 (67) | 8,063 |
| Lecithin and Acrylic Polymer | 6/9 (67) | 45,614 |

Anti-BSA IgG antibody titers were over five times higher when a combination of lecithin and acrylic polymer was used as adjuvant than when acrylic polymer was used alone and 323 times higher than when lecithin was used alone. This demonstrates that the combination of lecithin and acrylic polymer is far more effective at delivering the antigen orally to the mucosal surface for uptake by lymphoid tissue than either lecithin or acrylic polymer alone. Although, not all of the mice showed a serum anti-BSA IgG antibody response the results clearly show a synergistic adjuvant effect of lecithin combined with the acrylic polymer. However, the mice that did not seroconvert may have had a secretory IgA antibody response. Indeed, oral vaccination, and mucosal vaccination in general, stimulates IgA secreting cells at mucosal surfaces.

EXAMPLE 3

Second Test of Lecithin/Polymer Adjuvant by the Oral Route

Two vaccines were prepared in PBS that comprised the antigen, BSA Fraction 5, at a concentration of 400 $\mu$g per ml. One vaccine contained no adjuvant only BSA. The other vaccine was comprised of BSA adjuvanted with 3 mg/ml of lecithin and 3 mg/ml of acrylic polymer. The vaccine was assembled as described in Example 2. On days 0, 4, 27, and 31 groups of CF-1 female mice from Charles-River Laboratories and weighing approximately 18 grams, were administered 0.5 ml of vaccine orally by feeding needle. On day 52, 21 days post vaccination, mice were euthanized and bled by the brachial artery. End-point anti-BSA serum IgG antibody titers were determined by ELISA. A 1/100 starting dilution of serum was used due to non-specific background color development at dilutions less than 1/100. Results are recorded in Table 3:

TABLE 3

| Comparative Results of Adjuvant Versus Control | | |
|---|---|---|
| Adjuvant Composition | No. of Mice with Titer >/= 1/100 (%) | Reciprocal of Geometric Mean of Mice with Titers |
| None | 1/12 (8) | 100 |
| Lecithin and Acrylic Polymer | 9/11 (82) | 18,812 |

This study again demonstrates that the combination of lecithin and acrylic polymer is effective in delivering antigen to oral mucosal surfaces.

In a separate study, 4/10 mice that received this same vaccine had a geometric mean titer of 1/1,345 six weeks after only a single vaccination. This demonstrates the potential of the adjuvant composition, when once optimized, to engender an immune response of long duration.

EXAMPLE 4

Administration of Vaccine Intranasally

Two experimental vaccines for delivery by the intranasal route were prepared in PBS comprising the antigen, BSA, at a concentration of 500 $\mu$g/ml. One vaccine was comprised of BSA alone. The second vaccine was comprised of BSA adjuvanted with a combination of 3 mg/ml of lecithin and 3 mg/ml of the acrylic polymer. The lecithin and acrylic polymer were first mixed with a laboratory bench top magnetic stir bar and then in a Waring blender using an emulsification head. BSA was then added and mixed again using the emulsification head. Mice were vaccinated on days 0 and 20. Forty $\mu$l containing 20 $\mu$g of BSA antigen were placed on the nose while the mouth was held shut. The vaccine entered the nose when the mouse inhaled. On day 41, 21 days post second vaccination, the mice were euthanized and bled by cutting the brachial artery. Anti-BSA antibody titers were determined by ELISA. The starting dilution of serum was at 1/100 due to non-specific background color development at lower dilutions. Results are shown in Table 4:

TABLE 4

| Comparative Results of Adjuvant Versus Control | | |
|---|---|---|
| Adjuvant Composition | No. of Mice with Titer >/= 1/100 (%) | Reciprocal of Geometric Mean of Mice with Titers |
| None | 0/11 (0%) | 0 |
| Lecithin and Acrylic Polymer | 7/12 (58%) | 269 |

None of the mice (0/11) vaccinated with BSA alone seroconverted. The BSA antigen alone, when administered intranasally, failed to stimulate a serum antibody response in any of the mice. In contrast, 7 of 12 mice, or 58%, developed serum anti-BSA IgG antibody titers as high as 1/3200 following intranasal vaccination with BSA in combination with the invention comprised of lecithin and acrylic polymer. The fact that not all mice seroconverted suggests that not enough, or perhaps none of the vaccine was inhaled by those mice that did not have an antibody titer greater than 1/100. Indeed, some, perhaps most, of the vaccine was observed to run off the nose or was blown off the nose when the mouse exhaled. Still, the results of this study show that the invention, comprised of lecithin and acrylic polymer, functions effectively as an adjuvant for the intranasal delivery of a vaccine antigen.

EXAMPLE 5

Use of Adjuvant with Vaccine in Swine

The adjuvant invention comprising a combination of 2 mg/ml of lecithin and 2 mg/ml of acrylic polymer was used as a diluent for modified-live pseudorabies virus (ML-PRV) for swine. This adjuvant diluent and a control diluent consisting of sterile water were used to rehydrate lyophilized (ML-PRV). The ML-PRV was rehydrated immediately prior to vaccination. Groups of 10 weaned piglets, 6 weeks of age, were vaccinated on days 0 and 21. Blood serum was collected on days 2, 20, 28, and 48 for serological testing for anti-PRV serum neutralizing antibodies. The anti-PRV antibody responses of piglets in the different vaccine groups are shown in Table 5.

TABLE 5

| Results in Pigs | | | | |
|---|---|---|---|---|
| Adjuvant | Geometric Mean Virus Neutralizing Antibody Titer on Days Post First Vaccination | | | |
| Diluent | Day 2 | Day 20 | Day 28 | Day 48 |
| Non-Vaccinated Control | 0 | 0 | 0 | 0 |
| Water | 0 | 4 | 34 | 21 |
| Lecithin/ Arcylic Polymer | 0 | 6 | 69 | 52 |

This study showed that the invention comprising a lecithin and acrylic polymer combination functions as an adjuvant for a ML-virus vaccine adjuvant, in this case swine ML Pseudorabies vaccine virus. The virus neutralizing anti-PRV antibody titer to ML-PRV, which by itself is a very good antigen without an adjuvant and is used commercially without an adjuvant, was over twice as high when the lecithin/acrylic polymer was used instead of water.

EXAMPLE 6

Lecithin and Acrylic Copolymer Adjuvant Supports H1N1 and H3N2 Vaccine Responses

Lecithin and acrylic copolymer adjuvant as described and prepared herein was analyzed for its effectiveness at supporting the immunization of swine against H1N1 and H2N2 viral antigens. Inventive adjuvants herein were compared to a commercially available adjuvant, 5% Amphigen®, to identify the capacity of adjuvants as described herein to support viral antigen based vaccines. Each adjuvant was combined with H1N1 and H3N2 antigens (derived from a released lyophilized commercial product (FluSure™, Pfizer Animal Group). Test groups for vaccination included 29 to 35 day old piglets.
Four treatment groups were observed (each group having 15 piglets, except the negative control group which had 5 piglets): T1, 5% Amphigen, positive control; T2, Quil A alone adjuvant; T3, lecithin and acrylic copolymer of the invention, intramuscular; and T4, lecithin and acrylic copolymer of the invention, intranasal. T5 was a control group that was un-vaccinated. T1, T2 and T3 received one intramuscular dose on day 0, T4 received one intranasal dose on day 0.
Blood samples were obtained from each animal participating in the study on days 0, 21 and 35. Table 6 shows data from H1N1 titer, Table 7 shows data from the H3N2 titer:

TABLE 6: Number of Piglets with H1N1 Swine Influenza Titers and Geometric Mean Titers

| Treat No. | Test Art | Treat. Group | No. of Piglets With H1N1 Titers/Total Piglets | | | H1N1 Geometric Mean Titers | | |
|---|---|---|---|---|---|---|---|---|
| | | | Day 0 | Day 21 | Day 35 | Day 0 | Day 21 | Day 35 |
| T1 | 1 | SIV-Kill Amphigen | 0/15 | 15/15 | 11/15 | 5.0 | 24.1 | 11.5 |
| T2 | 2 | SIV-Kill Quil A | 0/15 | 6/15 | 0/15 | 5.0 | 6.9 | 5.0 |
| T3 | 3 | SIV-Kill LAP | 0/15 | 15/15 | 13/15 | 5.0 | 24.1 | 14.5 |
| T4 | 4 | SIV-Kill LAP | 0/15 | 0/15 | 0/15 | 5.0 | 5.0 | 5.0 |
| T5 | 5 | Neg. Control | 015 | 015 | 015 | 5.0 | 5.0 | 5.0 |
| No. of piglets with H1N1 Titers - Piglets with titers of 10 or higher were determined as positive. Geometric Mean Titers - A titer of 5 was assigned to negative titers for calculation of geometric mean. SIV - Lyophilized swine influenza (H1N1, H3N2) virus killed viral vaccine (FluSure™, Pfizer Animal Health). LAP - Lecithin acrylic polymer as described in Examples 1-5. | | | | | | | | |

TABLE 7: Number of piglets with H3N2 Swine Influenza Titers and Geometric Mean Titers

| Treat No. | Test Art | Treat. Group | No. of Piglets With H3N2 Titers/Total Piglets | | | H3N2 Geometric Mean Titers | | |
|---|---|---|---|---|---|---|---|---|
| | | | Day 0 | Day 21 | Day 35 | Day 0 | Day 21 | Day 35 |
| T1 | 1 | SIV-Kill Amphigen | 11/15 | 15/15 | 15/15 | 14.5 | 20.9 | 16.6 |
| T2 | 2 | SIV-Kill Quil A | 10/15 | 10/15 | 3/15 | 15.2 | 8.3 | 5.7 |
| T3 | 3 | SIV-Kill LAP | 10/15 | 15/15 | 15/15 | 13.8 | 28.9 | 17.4 |
| T4 | 4 | SIV-Kill LAP | 12/15 | 6/15 | 1/15 | 16.6 | 6.9 | 5.2 |
| T5 | 5 | Neg. Control | 4/5 | 3/5 | 1/5 | 17.4 | 8.7 | 5.7 |

No. of piglets with H3N2 titers - Piglets with titers of 10 or higher were designated as positive for titers.
Geometric Mean Titers - A titer of 5 was assigned a negative titer for calculations of geometric mean titers.
SIV - Lyophilzed swine influenza (H1N1, H3N2) virus killed viral vaccine (FluSure™, Pfizer Animal Health).
Amphigen® - Amphigen® adjuvant (Pfizer Animal Health)
LAP - Lecithin acrylic polymer as described in Examples 1-5.

Referring to Tables 6 and 7, the results indicate lecithin and acrylic copolymer adjuvants of the invention induce significant serological responses to H1N1 and H3N2 swine influenza viruses in piglets. The responses were similar to conventional commercial adjuvants and significantly better than a Quil A alone adjuvant. The data in Example 6 further show the utility of the lecithin/acrylic polymer adjuvants of the invention and show that additives alone, for example Quil A, provide for a minimal immunological response under identical conditions.

EXAMPLE 7

Immune Response to Vaccine Enhanced by Inclusion of Additives

The immunogenecity of a lecithin, acrylic polymer, Quil A and cholesterol adjuvant of the invention was tested against Amphigen, a commercial adjuvant. The study was performed to determine the effectiveness of eliciting an enhanced immune response through inclusion of cholesterol and Quil A in adjuvant embodiments described herein.

Adjuvant was prepared as described above, except in this Example, 15 mg/ml lecithin was combined with 10 mg/ml acrylic polymer. Each vaccine dose included 1.25 mg of adjuvant (as compared to 5 mg of Amphigen).

The adjuvant samples were spiked with 0.5 mg/ml of Quil A/cholesterol. All adjuvant samples further received $50\mu g$, $5\mu g$ or $0.5\ \mu g$ AIV-HA antigen. Ingredients described above were combined as discussed herein to provide 3 different antigen concentrations for each lecithin/acrylic polymer/additive sample and Amphigen seample. All samples were stored at 4°C.

Twenty-nine to thirty-five day old piglets were vaccinated and bleeds taken on days one, fourteen and twenty eight. Titers were determined for each condition and mean HAI titer determined.

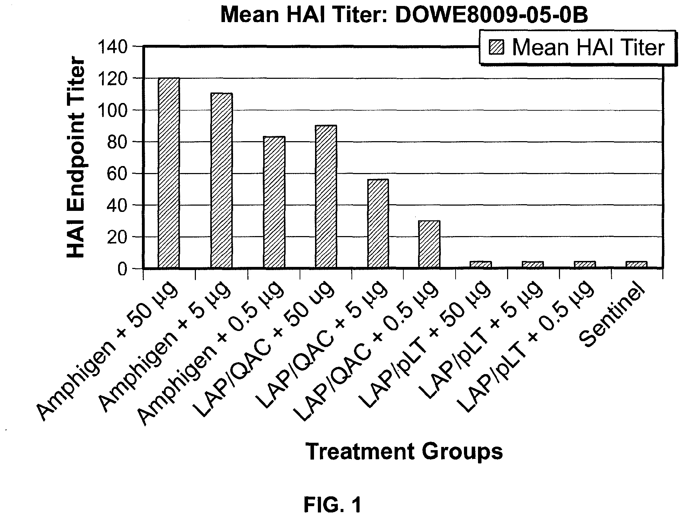

As shown in Figure 1, the study showed a surprising increase in mean HAI titer with cholesterol and Quil A included in the adjuvant and therefore vaccine preparation. In each case, the increase in titer was antigen concentration dependent and showed a significant increase comparable to corresponding antigen spiked Amphigen samples. It is noted that the amount of lecithin and acrylic polymer was limited (1.25 mg) to provide a more sensitive platform for identifying effects of the inventive adjuvants described herein. The low dose lecithin/acrylic polymer/additive adjuvant provided an adequate platform for analyzing the adjuvant's capacity to elicit an immune response, which was comparable to a full dose Amphigen based adjuvant.

EXAMPLE 8

Lecithin and Acrylic Polymer Adjuvant is Facilitated By Inclusion of Additives

The immunogenecity of a lecithin, acrylic polymer, cholesterol, and Quil A adjuvant was tested in chickens. Cell line based antigen was tested in this manner in order to determine utility of adjuvants (additive based) in accordance with the present invention.

Adjuvant was prepared using 33.5 μg/dose Quil A, 32.4 μg/dose cholesterol, 800 μg/dose lecithin and 500 μg/dose acrylic copolymer 974PNF. Vaccine antigen was a stably transfected cell line using a H5 HA expressing plasmid (CHO-HA-10). In some vaccines, an antigen stabilizing agent was added to the material, i.e., *Nicotiana tabacum* cell line lysate.

Sixty five pathogen-free Leghorn chickens (10 day old) were obtained and quarantined for nine days prior to start-up of the experiment. Birds were split into eleven groups (six birds per group for groups T1 through T10 and five birds for T11). Each group was housed together. Treatment group T11 birds were a baseline group and used to obtain a Day 0 bleed. Study design is provided in Table 8:

TABLE 8: Example 8 study design

| No. | Treatment Group | Processing Method | No. of Birds | Vacination | | | Bleed Days* |
|---|---|---|---|---|---|---|---|
| | | | | Days | Dose | Route | |
| T1 | CHO lys. NT-1 ext Adjuvant | Mixing | 6 | 0, 14 | 0.5 ml | SQ | 14, 28 |
| T2 | CHOHA0102 Adjuvant | Mixing | 6 on zero 5 on 14 | 0, 14 | 0.5 ml | SQ | 14, 28 |
| T3 | CHOHA0102 Adjuvant | Microfluid. Non-Clarified | 6 | 0, 14 | 0.5 ml | SQ | 14, 28 |
| T4 | CHOHA0102 Adjuvant | Microfluid. Clarified | 6 | 0, 14 | 0.5 ml | SQ | 14, 28 |
| T5 | CHOHA0102 Adjuvant | Silverson Non-Clarified | 6 | 0, 14 | 0.5 ml | SQ | 14, 28 |
| T6 | CHOHA0102 NT-1 ext Adjuvant | Mixing | 6 | 0, 14 | 0.5 ml | SQ | 14, 28 |
| T7 | CHOHA0102 NT-1 ext Adjuvant | Microfluid. Non-Clarified | 6 | 0, 14 | 0.5 ml | SQ | 14, 28 |
| T8 | CHOHA0102 NT-1 ext Adjuvant | Microfluid. Clarified | 6 | 0, 14 | 0.5 ml | SQ | 14, 28 |
| T9 | CHOHA0102 NT-1 ext Adjuvant | Silverson Non-Clarified | 6 | 0, 14 | 0.5 ml | SQ | 14, 28 |
| T10 | H5N9 AIV Adjuvant | Mixing | 6 | 0, 14 | 0.5 ml | SQ | 14, 28 |
| T11 | Baseline Bleed | NA | 5 | NA | NA | NA | 0 |

Bleed days - serum evaluated by hemagglutination inhibition with A/Turkey/Wisconsin/68 (H5N9)

CHO lysate - Non-transfected CHO cells

NT-1 *extract - Nicotiana tabacum,* lyophilized, non-transformed clarified cell lysate

Adjuvant - Lecithin, acrylic copolymer, Quil A, and cholesterol

SQ - subcutaneous administration

CHOHA0102 - CHO cells transfected with H5 hemagglutinin

H5N9 AIV - Inactivated HSN9 avian influenza virus A/Turkey/Wisconsin/68

NA - not applicable

As shown in Table 9, all vaccinated treatment groups (T2-T9) were clearly different from the negative control (T1). Although slight differences were observed, little differentiation was discernable between treatment groups that did or did not receive the *Nicotiana tabacum* cell line lysate or between the various processing methods.

TABLE 9: Seroconversion rates and avian influenza hemagglutination inhibition geometric mean titer results

| Treatment group | Treatment Group | | No. of Birds Seroconverting/Tot. Birds | | Avian Influenza Geometric Mean Titers | |
|---|---|---|---|---|---|---|
| | Antigen Ad | Processing | Day 14 | Day 28 | Day 14 | Day 28 |
| T1 | CHO lys. NT-1 ext Adjuvant | Mixing | 0/6 | 0/6 | 4.0 | 4.0 |
| T2 | CHOHA0102 Adjuvant | Mixing | 2/6 | 5/5 | 8.0 | 48.5 |
| T3 | CHOHA0102 Adjuvant | Microfluid. Non-Clarified | 5/6 | 5/6 | 11.3 | 22.6 |
| T4 | CHOHA0102 Adjuvant | Microfluid. Clarified | 3/6 | 5/6 | 9.0 | 28.5 |
| T5 | CHOHA0102 Adjuvant | Silverson Non-Clarified | 4/6 | 4/6 | 14.3 | 22.6 |
| T6 | CHOHA0102 NT-1 ext Adjuvant | Mixing | 4/6 | 5/6 | 10.1 | 32.0 |
| T7 | CHOHA0102 NT-1 ext Adjuvant | Microfluid. Non-Clarified | 3/6 | 5/6 | 10.1 | 18.0 |
| T8 | CHOHA0102 NT-1 ext Adjuvant | Microfluid. Clarified | 3/6 | 6/6 | 9.0 | 20.2 |
| T9 | CHOHA0102 NT-1 ext Adjuvant | Silverson Non-Clarified | 2/6 | 5/6 | 5.7 | 25.4 |
| T10 | H5N9 AIV Adjuvant | Mixing | 3/6 | 6/6 | 8.0 | 228.1 |

Results from the Example show that additive based adjuvants of the invention support a cell line based antigen, i.e., a cell line that expresses the antigen, and provided excellent immunogenicity in chickens. This data further supports the conclusion that Quil A and cholesterol when used in combination with other adjuvant based embodiments described herein have surprising utility in the context of the present invention.

EXAMPLE 9:

Micrograph Data: Embodiments of the Present Invention Have Net-Like Structure

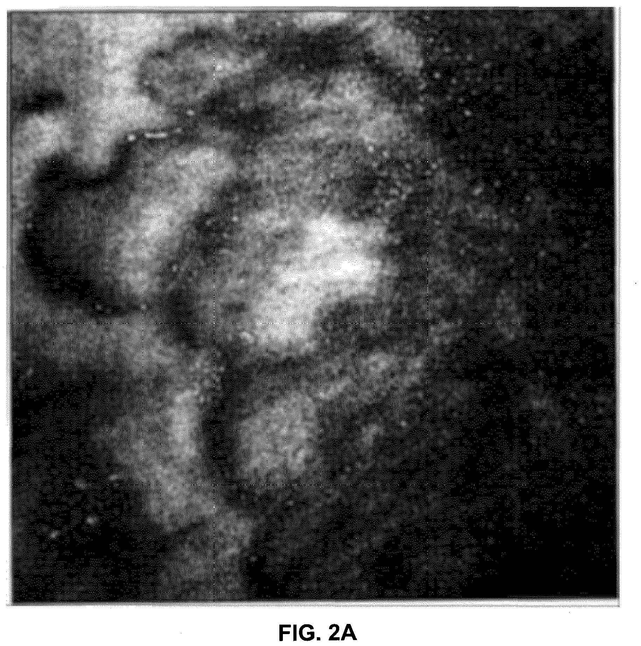

Inventive adjuvant compositions were as prepared and described in previous Examples. Adjuvant samples were visualized by transmission electron microscopy. Adjuvants included lecithin and polymer, no additives, and were sterilized by autoclaving. An illustrative micrograph at 30,000X magnification is shown in Figure 2A.

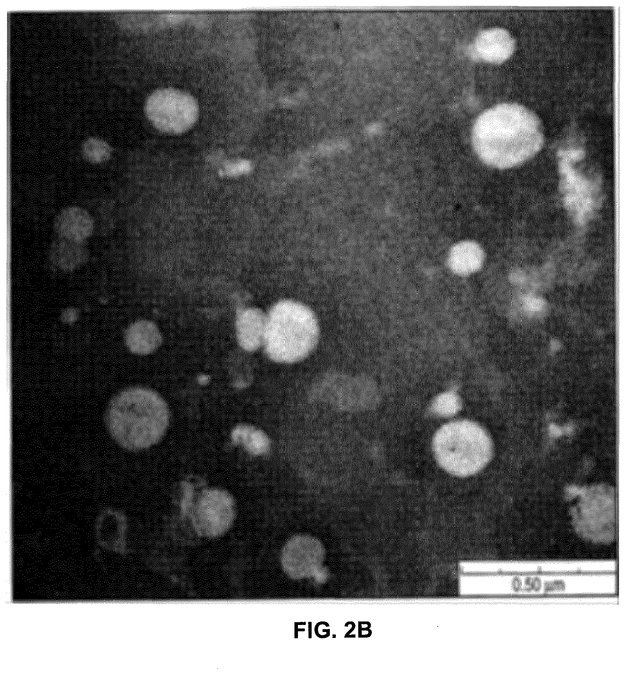

For comparison, an illustrative emulsion as prepared by the methods described in US Patent No. 5,716,637 (Anselem et al.), and visualized via transmission electron microscopy at the same magnification (30,000X) is shown in Figure 2B. As described in the description of the Anselem patent, the adjuvant was microfiltered and not autoclaved.

The adjuvant prepared via the methods of US Patent No. 5,716,637, show an expected emulsion structure of lipid droplets in an aqueous phase. In contrast, the micrograph shown in Figure 2 A illustrates that adjuvants of the present invention have a significantly different physical structure or distribution than the adjuvants described in Anselem. Adjuvants of the present invention show a diffuse net-like structure with significant polymer content combining with the lecithin (phospholipids) to provide the unexpected structure of the present invention. This is a surprising given the significant difference is structure between the two adjuvant compositions.

EXAMPLE 10

Calcium Phosphate Facilitates Immunity of DNA-Based Vaccine

Lecithin and acrylic copolymer adjuvant as described and prepared herein was analyzed alone and in combination with calcium phosphate ($CaPO_4$) to determine effectiveness at supporting the immunization of chicks against an avian influenza DNA antigen (H5N9 AIV HA DNA). Inventive adjuvants herein were compared to a commercially available adjuvant, having the same antigen. The commercially available adjuvant was also tested with $CaPO_4$.

Chick treatment groups were immunized on day 0 and day 16. Serum testing was performed on each treatment group and HAI titers determined using standard assays.

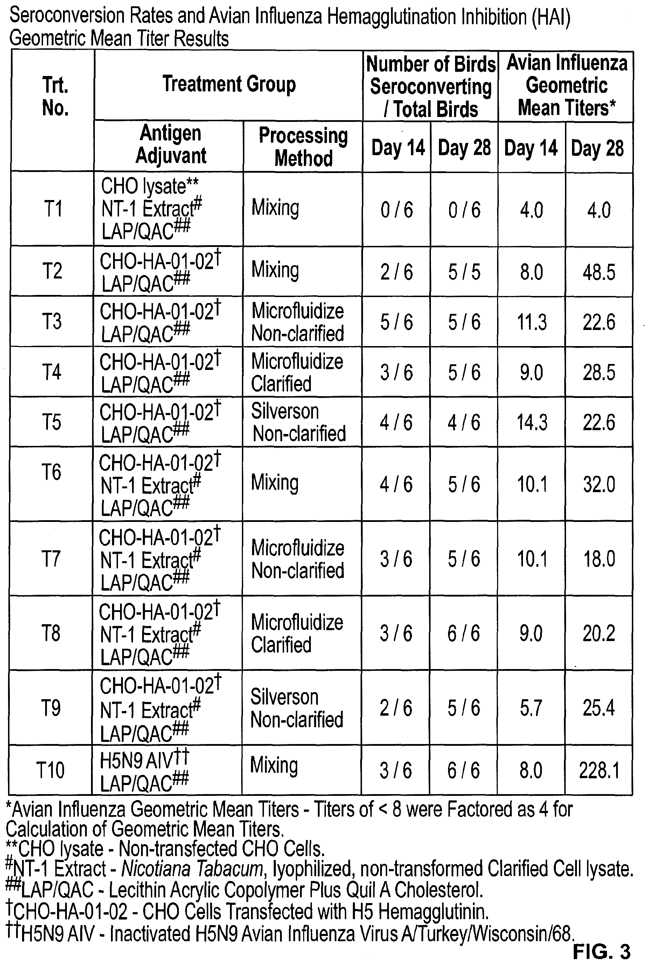

Figure 3 shows data from HAI GMT serum titers for adjuvant only, no antigen (Amphigen®); antigen only, no adjuvant; Amphigen®; Amphigen® with $CaPO_4$; Lecithin and Copolymer; and Lecithin, Copolymer and $CaPO_4$. The results in Figure 3 indicate that the lecithin, copolymer and calcium phosphate group vaccine provided significantly higher levels of immunity than lecithin and copolymer alone or Amphigen with or without calcium phosphate.

This combination of lecithin, copolymer and calcium phosphate shows an unexpected capacity to transform poorly immunogenic DNA vaccines into highly effective vaccines.

EXAMPLE 11

Enhanced Potency of FMD Vaccine

Experimental design: Second generation human adenovirus type 5 (Ad5) vector for FMDV serotype A24 Cruzeiro administered to pigs subcutaneously at two sites with or without adjuvant. The animals were challenged 21 days post-vaccination at doses 10-fold higher than recommended.

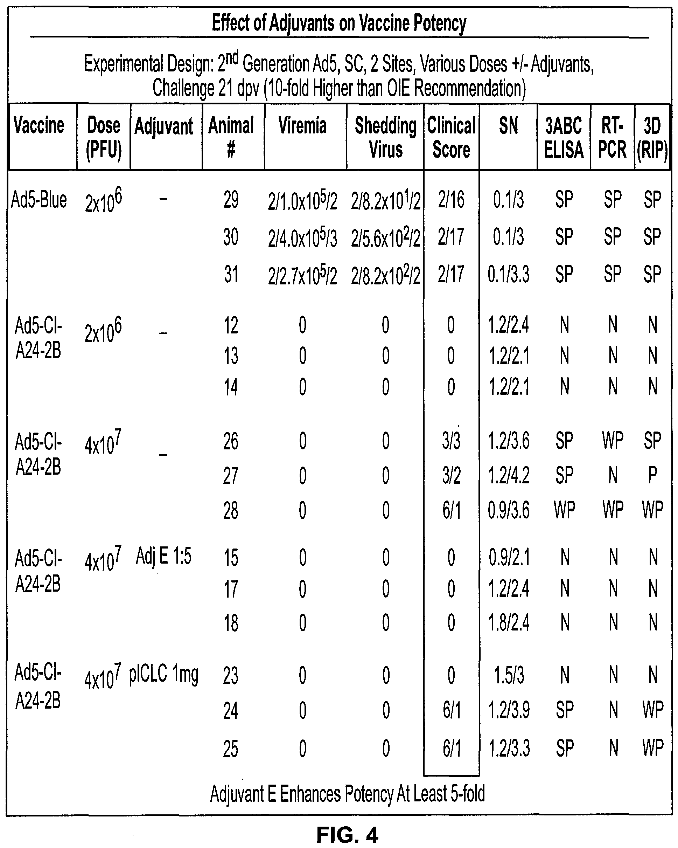

Figure 4 shows the results of the experimental Adenovirus vector-based FMD vaccine alone or in combination with one of two adjuvants: a DNA plasmid called pICLC or Adjuvant E (Adjuplex/Vetplex with Quil A and Cholesterol). Adjuvant E increased potency by at least 5-fold whereas the pICLC adjuvant was less effective. This data further supports the conclusion that Quil A and cholesterol when used in combination with other adjuvant based embodiments described herein have surprising utility in the context of the present invention.

EXAMPLE 12

Effect of Vaccine Adjuvants on Adeno-Vector Viruses

The overarching goal of this research was to produce and evaluate Foot-and-mouth disease (FMD) single or combinational vaccines comprising replication-defective recombinant human adenovirus carrying (a) the FMD VP1 capsid and 3C protease coding regions and/or (b) bovine or porcine type 1 interferon genes. This strategy is being used to develop next generation molecular FMD licensed vaccines for stock piling by the National Veterinary Stockpile Program for use as an FMD countermeasure in emergency outbreaks.

One research area of this project was the addition of vaccine adjuvants to the adeno-based vector vaccines for enhancement of their immunogenecity and efficacy. Two adjuvants (Adjuplex-LAP and Adjuplex-LE) were evaluated in vitro for virucidal effects on the vaccine vectors under different time and temperature conditions. The conclusion drawn from this study was that Adjuplex LAP did not have any virucidal effect on Ad5bIFN$\alpha$ virus at the recommended or 4-fold concentration level. The incubation temperatures and times for these studies were 20° C, or 39° C, and 1 or 24 hours, respectively. However, similar results were not observed for Adjuplex-LE. No titer reductions were observed at 39° C and one hour incubation; however when the incubation was increased to 24 hours, there were significant reductions in virus titers at both concentrations. Results from this study would suggest that Adjuplex-LE adjuvant produced a virucidal effect on Ad5-bIFN$\alpha$ virus at both the recommended and two-fold higher concentration levels.

Similar results were obtained with Ad5-O1 virus when it was mixed with both Adjuplex LAP and LE, respectively, at the recommended quantity and when the amount was increased two- or four-fold. Furthermore, there were no significant titer reductions even when the mixtures were incubated at $39^0$ C for 24 hours. The $39^0$ C incubation temperature was selected because that is the average cattle rectal body temperature. It is recommended that Adjuplex LAP should be the adjuvant of choice to be combined with the Ad5-bIFN$\alpha$, Ad5-O1 and other Ad5 based FMD sub unit vaccines for clinical evaluation in cattle and pigs.

Foot-and-mouth disease (FMD) is an economically important and highly contagious viral disease of cloven-hoofed livestock and wildlife including cattle, swine, sheep, goats, and deer. The recent re-emergence of FMD in both developing and developed nations has refocused world's attention on universe control strategies particularly in the USA.

In many countries the control and eradication of FMD are by immunization of susceptible animals using commercially available FMD vaccines, which are based on conventional chemically inactivated vaccines emulsified with adjuvants. Failure to completely inactivate the vaccine has led to outbreaks of the disease. There is no approved diagnostic test available to reliably differentiate vaccinated from infected animals. Furthermore vaccinated animals can become disease carriers following contact with FMD virus. These disadvantages of inactivated whole FMD vaccine have made FMD-free countries to be reluctant to vaccinate their livestock during outbreaks.

In order to overcome some of the problems associated with convectional FMD vaccines, many approaches have been utilized to develop alternative FMD vaccines, including construction of modified live-virus, biosynthetic proteins, synthetic peptides, naked DNA vectors, and recombinant viruses. The use of human adenovirus as a vector for FMD vaccines has been met with variable results, sometimes resulting in incomplete protection or failure of vaccinated animals to develop a neutralizing antibody.

In this study, two adjuvants viz Adjuplex-LAP and Adjuplex-LE were investigated in vitro as possible adjuvants for Ad5 FMD subunit vaccines. This was a preliminary investigation prior to in vivo studies. Experiments were set up to determine both their cytotoxic effect on 293 cells and their virucidal effects on Ad5-FMD viruses.

Adjuplex-LAP is a mucosal vaccine adjuvant as well as an adjuvant for parenterally administered vaccines. The adjuvant is a lecithin phospholipid/acrylic polymer combination. The combination forms a mucoadhesive matrix that facilitates the adsorption of vaccine antigens to mucosal surfaces and subsequent absorption and presentation of antigen to cells of the immune system. Both adjuvant components are used in the pharmaceutical and biological industries and thus have value as a delivery and adjuvant system for vaccine antigens.

Adjuplex-LE is a 5%, or less, oil-in-water emulsion, the oil droplets which are covered by lecithin derived phospholipid vesicles. The lipid vesicles act as a carrier for vaccine antigens and make them accessible to cells of the immune system. The lipid vesicles on the surface of the oil droplets is also a safety feature making the oil less irritable or not irritable at all to tissue at the injection site. The adjuvant is also non-virucidal. Therefore the formulation can be, and is, used to adjuvant modified-live virus vaccines. The adjuvant can be mixed directly with vaccine antigens without further emulsification or the antigens can be added at the time of emulsification.

The redox indicator Alamar Blue™ (AB), a fluorescent dye, which has been used in mammalian cell culture cytotoxicity assays. AB is a safe, nontoxic aqueous dye, which is used to assess cell viability and cell proliferation because it is stable in cell culture. It has also been shown to be a rapid and simple nonradioactive assay alternative to the [$^3$H] thymidine incorporation assay. AB both fluoresces and changes color in response to chemical reduction, and the extent of the conversion is a reflection of cell viability. AB assay is a simple, one-step procedure. Alamar Blue™ assay was set up to study the cytotoxicity effects of two vaccine adjuvants 293 cells.

There were two objectives: (a) evaluate the relative 293 cell cytotoxity of vaccine adjuvants in combination with Ad5-bovine interferon alpha (Ad5bIFN $\alpha$) and Ad5O1 vectors, and (b) establish if the vaccine adjuvants are virucidal for Ad5bIFN$\alpha$ vector.

Alamar Blue™ assay was set up to study the cytotoxicity effect of vaccine adjuvants on 293 cells. Alamar Blue™ is a safe, nontoxic aqueous dye which is used to assess cell viability and cell proliferation.

MATERIALS AND METHODS

*293 Cells*

Human embryonic kidney (293) cells were obtained from Dr. Patrick Hearing, Department of Microbiology, Stony Brook University, Stony Brook, NY, and were propagated in minimum essential medium (MEM) containing 10% fetal bovine serum (FBS), 1% antibiotic-antimycotic solution, and 1% MEM non essential amino acid (NEAA). 293 cells of passages 15 and 36 were used for transfection, propagation of viruses, virus titrations and performing of cytotoxicity assays.

*Ad5bIFN$\alpha$ and Ad5O1 plasmids*

The two plasmids were provided by Dr. Laszlo Zsak from US Department of Homeland Security, Targeted Advanced Development, Plum Island Animal Diseases Center, Orient Point, NY.

*Transfection of Ad5bIFN$\alpha$ and Ad5O1 plasmids in 293 cells*

The pAd5bIFN$\alpha$ and pAd5O1 were linearized by digestion with restriction enzyme PacI and transfected in 293 cells using Lipofectamin™2000.

*Production and Purification of AdSbIFNα and Ad5O1 viruses (vaccine vectors)*

The 2 viruses were harvested with the appearance of the initial plaques, which were then grown in large quantities in 293 cells, and purified utilizing a nonlinear followed by a linear CsCl gradient centrifugation.

*Dilutions of vaccine viruses (Ad5bIFNα and Ad5O1)*

1:10, 1:100 and 1:100 dilutions of both Ad5bIFNα and Ad5O1 vaccine viruses were prepared respectively in EMEM containing 2% FBS, 1% antibiotic-antimycotic solution, and 1% MEM-NEAA.

*Dilutions of adjuvants (Adjuplex-LAP and Adjuplex-LE)*

1:20, 1:200, 1:2000, and 1:20,000 dilutions of both Adjuplex-LAP and Adjuplex-LE adjuvants were prepared respectively in EMEM containing 2% FBS, 1% antibiotic-antimycotic solution, and 1% MEM-NEAA.

*Alamar Blue (AB)*

AB was aliquoted and stored at -80° C. Prior to each experiment, AB was brought to room temperature and vortexed. Exposure of AB to light was minimized throughout the experiments.

*Alamar Blue (AB) cytotoxicity assay (Cell viability assay)*

Cell viability of 293 cells was assessed by AB cytotoxicity assay. 293 cells in EMEM containing 2% FBS, 1% antibiotic-antimycotic solution, and 1% MEM-NEAA were seeded at a density of $1 \times 10^6$ viable cells/ml ($1 \times 10^5$/well) in a 96 well Treatment (BD Falcon* Primaria* Tissue culture Treatments, Fisher Scientific Company, Suwanee, GA). In a second plate, cells were seeded at a density of $10 \times 10^6$ viable cells/ml ($1 \times 10^6$/well) to determine the optimal cell concentration for the cytotoxicity study.

Table 10 shows the addition of diluted Ad5bIFNα, Ad5O1, Adjuplex-LAP and Adjuplex-LE to the cells. Briefly, 0.1 ml of each of the dilutions of Ad5bIFNα and Ad5O1 (1:10, 1:100, 1:100) was added to each well respectively in triplicates. 0.1 ml of each of the dilutions of the adjuvants (1:20, 1:200, 1:2000, and 1:20,000) was added to each well respectively in triplicates. EMEM containing 2% FBS, 1% antibiotic-antimycotic solution, and 1% MEM-NEAA was added to wells A1, A2, A3, D1, D2 and D3 as negative controls.

The two treatments were incubated at 37° C in a 5% $CO_2$ atmosphere for approximately 18 hours, after which 20 μl AB was added to each well. The treatments were returned to the incubator.

Optical densities (OD) at 570 nm and 600 nm were measured with the $EL_X808$ ultra microplate reader (BioTek Instruments, Inc., Winooski, VT) at approximately 42 hours, 50 hours, and 68 hours (total culture times).

The OD data were analyzed as follows: (a), determine % difference in reduction of AB (between media growth control wells and treatment wells); this will indicate the amount inhibition (or stimulation) of cell growth in treatment wells with respect to media control wells, and (b). determine % reduction of AB in media control and in treatment wells; this will indicate the amount of cell growth in media control and treatment wells. Treatments with higher % reduction than media controls are considered to stimulate cell growth. Treatments with lower % reduction than media controls are considered cytotoxic. Treatments with the same % reduction are neither cytotoxic nor stimulatory.

*Calculation of Alamar Blue (AB) reduction*

Percent reduction of AB was calculated using the manufacturer's formula (33). In monitoring AB reduction spectrophotometrically, reduction is expressed as a percentage (% reduced).

The calculation of % Reduced is as follows when the samples are read at

$\lambda 1 = 570$ nm
$\lambda 2 = 600$ nm

$$\% \text{ Reduced} = \frac{(\varepsilon ox\ \lambda 2)\ (A\ \lambda 1) - (\varepsilon ox\ \lambda 1)\ (A\ \lambda 2)}{(\varepsilon red\ \lambda 1)\ (A'\ \lambda 2) - (\varepsilon red\ \lambda 2)\ (A'\ \lambda 1)} \times 100$$

Where:

($\varepsilon$red $\lambda$1) = 155,677 (Molar extinction coefficient of reduced alamarBlue™ at 570 nm)
($\varepsilon$red $\lambda$2) = 14,652 (Molar extinction coefficient of reduced alamarBlue™ at 600 nm)
($\varepsilon$ox $\lambda$1) = 80,586 (Molar extinction coefficient of oxidized alamarBlue™ at 570 nm)
($\varepsilon$ox $\lambda$2) = 117,216 (Molar extinction coefficient of oxidized alamarBlue™ at 600 nm)
(A $\lambda$1) = Absorbance of test wells at 570 nm
(A $\lambda$2) = Absorbance of test wells at 600 nm
(A'$\lambda$1) = Absorbance of negative control wells which contain medium plus alamarBlue™ but to which no cells have been added at 570 nm.
(A'$\lambda$2) = Absorbance of negative control wells which contain medium plus alamarBlue™ but to which no cells have been added at 600 nm.

In reporting alamarBlue™ reduction by monitoring absorbance, data are expressed as percent alamarBlue™ reduced as a function of time of incubation. The AB assay was used to determine viability of 293 cells to vaccine adjuvants.

*Virucidal Assay (TCID$_{50}$ Assay)*

The TCID$_{50}$ assay was employed to ascertain and measure if there were virucidal effects of Adjuplex-LAP and Adjuplex-LE adjuvants on Ad5bIFN$\alpha$ and Ad5O1 viruses.

293 cells were harvested from a T-150 flask of fresh 293 cells, and counted on a hemocytometer. A dilution of the cell suspension at $1 \times 10^5$/ml in MEM containing 2% FBS, 1% antibiotic-antimycotic solution, and 1% MEM-NEAA was made, and at least 10 ml was prepared for each 96 flat-bottomed well tissue culture plate.

Using a 12-channel pipette and a multichannel pipetter basin, 100 $\mu$l of the cell dilution ($10^4$ cells/per well) was seeded into 96-well tissue culture plates, and covered. They were incubated at $37^0$ C in a $CO_2$ incubator until used.

Frozen vials of Ad5bIFN$\alpha$ and Ad5O1 viruses were thawed and kept on ice at all times.

MEM supplemented with 2% FBS, 1% antibiotic-antimycotic solution, and 1% MEM-NEAA was used to make virus dilutions of $10^{-1}$ to $10^{-13}$. The 10 fold dilutions were made in 5 ml sterile, disposable tubes.

The cells were infected by adding 0.1 ml per well of each virus-adjuvant dilution immediately after the dilutions were made.

AB assay with a cell concentration of $10 \times 10^6$/ml was also set up.

*Preparation of Antigen-Adjuvant Mixtures*

*Ad5bIFN$\alpha$ virus-Adjuvant Mixtures*

Each of the two adjuvants was mixed with Ad5bIFN$\alpha$ according to the manufacturer recommended ratios (LAP: virus ratio, 1:4; LE: virus ratio, 1:1). In addition to the recommended ratios, additional ratios (LAP: antigen ratio, **4:1;** LE: antigen ratio, **2:1**) were also tested to increase the chances of having virucidal effects. Each mixture was made in a sterile 1 ml microtube and vortexed three times to ensure adequate mixing before use, and was incubated according to the appropriate conditions.

The media-virus mixture served as a control. The media consisted of MEM supplemented with 2% FBS, 1% antibiotic-antimycotic solution, and 1% MEM-NEAA. The ratios tested were as follows:

*Experiment 1*

Treatment A: 25$\mu$l Media + 100$\mu$L Ad5bIFN$\alpha$
Treatment B: 25$\mu$L LAP + 100$\mu$L Ad5bIFN$\alpha$
Treatment C: 100$\mu$L Media + 100$\mu$L Ad5bIFN$\alpha$
Treatment D: 100$\mu$L LE + 100$\mu$L Ad5bIFN$\alpha$

The mixtures were not incubated before dilutions were made.

*Experiment 2*

Treatment A: 25$\mu$l Media + 100$\mu$L Ad5bIFN$\alpha$

Treatment B: 25μL LAP + 100μL Ad5bIFNα
Treatment C: 100μL Media + 100μL Ad5bIFNα
Treatment D: 100μL LE + 100μL Ad5bIFNα

The mixtures were incubated at room temperature (20 - 25° C) for one hour before dilutions were made.

*Experiment 3*

Treatment A: 100 μl Media + 100μL Ad5bIFNα
Treatment B: 100 μL LAP **(4X)** + 100μL d5bIFNα
Treatment C: 100 μL LE + 100μL Ad5bIFNα
Treatment D: 200 μL Media + 100 μL Ad5bIFNα
Treatment E: 200 μL LE **(2X)** + 100 μL Ad5bIFNα

The mixtures were incubated at 39° C for one hour before dilutions were made.

*Experiment 4*

Treatment A: 100 μl Media + 100μL Ad5bIFNα
Treatment B: 100 μL LAP **(4X)** + 100μL Ad5bIFNα
Treatment C: 100 μL LE + 100μL Ad5bIFNα
Treatment D: 200 μL Media + 100 μL Ad5bIFNα
Treatment E: .200 μL LE **(2X)** + 100 μL Ad5bIFNα

The mixtures were incubated at 39° C for 24 hours before dilutions were made.

*Ad5O1-Adjuvant Mixtures*

Each of the two adjuvants was mixed with Ad5O1 according to the manufacturer recommended ratios (LAP: virus ratio, 1:4; LE: virus ratio, 1:1). In addition to the recommended ratios, additional ratios (LAP: virus ratio, **4:1;** LE: virus ratio, **2:1**) were also tested to increase the chances of having virucidal effects. Each mixture was made in a sterile 1 ml microtube and vortexed three times to ensure adequate mixing before use.

The media-virus mixture served as a control. The media consisted of MEM supplemented with 2% FBS, 1% antibiotic-antimycotic solution, and 1% MEM-NEAA. The ratios tested were as follows:

*Experiment 1*

Treatment A: 25μl Media + 100μL Ad5O1
Treatment B: 25μL LAP + 100μL Ad5O1
Treatment C: 100μL Media + 100μL Ad5O1
Treatment D: 100μL LE + 100μL Ad5O1

The mixtures were not incubated before being dilutions were made.

*Experiment 2:*

Treatment A: 25μl Media + 100μL Ad5O1
Treatment B: 25μL LAP + 100μL Ad5O1
Treatment C: 100μL Media + 100μL Ad5O1
Treatment D: 100μL LE + 100μL Ad5O1

The mixtures were incubated at room temperature (20 - 25$^0$ C) for one hour dilutions were made.

*Experiment 3*

Treatment A: 100 µl Media + 100µL Ad5O1
Treatment B: 100 µL LAP **(4X)** + 100µL Ad5O1
Treatment C: 100 µL LE + 100µL Ad5O1
Treatment D: 200 µL Media + 100 µL Ad5O1
Treatment E: 200 µL LE **(2X)** + 100 µL Ad5O1

The mixtures were incubated at 39$^0$ C for one hour before dilutions were made.

*Experiment 4*

Treatment A: 100 µl Media + 100µL Ad5O1
Treatment B: 100 µL LAP **(4X)** + 100µL Ad5O1
Treatment C: 100 µL LE + 100µL Ad5O1
Treatment D: 200 µL Media + 100 µL AdO1
Treatment E: .200 µL LE **(2X)** + 100 µL Ad5O1

The mixtures were incubated at 39° C for 24 hours before dilutions were made.

*Preparation of virus-adjuvant dilutions*

From each treatment, serial 10 fold dilutions of adjuvant-virus mixtures were prepared in 5 ml sterile, disposable tubes using MEM supplemented with 2% FBS, 1% antibiotic-antimycotic solution, and 1% MEM-NEAA to prepare adjuvant-virus dilutions of $10^{-1}$ to $10^{-13}$. 0.9 ml MEM supplemented with 2% FBS, 1% antibiotic-antimycotic solution, and 1% MEM-NEAA was dispensed into the first two tubes. To each of the eleven other tubes was added 1.8 ml.
0.1 ml of each adjuvant-virus mixture from each treatment was added to the first tube and mixed by votexing. Filtered pipette tips were changed between dilutions. 0.1 ml of the $10^{-1}$ dilution was withdrawn and transferred to the second tube containing MEM supplemented with 2% FBS, 1% antibiotic-antimycotic solution, and 1% MEM-NEAA.
0.2 ml of the $10^{-2}$ dilution was withdrawn and transferred to the third tube containing MEM supplemented with 2% FBS, 1% antibiotic-antimycotic solution, and 1% MEM-NEAA, and this became $10^{-3}$ virus dilution. The above steps were repeated to prepare the next virus dilutions ($10^{-4}$ to $10^{-13}$).

*Infection of Cells with Virus-Adjuvant Dilutions*

One 96-well plate was used for infection per treatment. Immediately after the dilutions were made, the cells were infected by adding 0.1 ml per well of each virus-adjuvant dilution. 0.1 ml of the virus-adjuvant suspension with the highest dilution was dispensed in column 1 wells to infect the cells in the 8 wells of this column.
The cells in the 8 wells of the next column; column 2 of the 96-well were infected with 0.1 ml of the next adjuvant-virus dilution ($10^{-12}$). The cells in the 8 wells of columns 3 through 11 were infected with 0.1 ml of the remaining virus-adjuvant dilutions ($10^{-11}$-$10^{-13}$). Pipette tips were changed between dilutions.
To test the cell viability and as a negative control (no adjuvant-virus control), 0.1 ml/well of MEM containing 2% FBS, 1% antibiotic-antimycotic solution and 1% MEM-NEAA was added to each well in column 12. Each plate was covered and incubated at 37° C in a $CO_2$ incubator for 10 days. Cells in each plate were observed daily by an inverted microscope for cytopathic effects (CPE) over the next 10 days. Observable CPE per column were counted and recorded. Final reading of each plate was done on the 10$^{th}$ day post incubation to determine the titer. A well was counted as positive even if only a small spot or a few cells showed CPE. The negative control wells were used for comparison. The test was valid if the negative controls did not show any CPE or cell growth problems, and the lowest dilution showed 100% infection (8/8) while the highest dilutions showed 0% infection (0/8).
The results were recorded for day 10, scoring wells "+" (CPE positive) or "-" (CPE negative) using the CPE scoring form in appendix A, and the ratio of positive wells per column was determined, and recorded it as in appendix A. After recording the assay data, the plates were placed in a biohazard bag and autoclaved and discarded as biohazardous waste.

For each plate (treatment), $TCID_{50}$/ml titer was calculated using the KÄRBER statistical method. Compare the $TCID_{50}$/ml between the treatments in each experiment to determine if there was a difference. A difference in titers between treatments in each experiment that is greater than 0.7 log was considered to be significant, which means the adjuvant in that experiment was virucidal to the tested virus.

RESULTS

*Alamar Blue (AB) cytotoxicity assay (Cell viability assay)*

Alamar Blue™ was used to measure 293 cell viability at two densities There was cell clumping at a density of $10 \times 10^6$ /ml for all treatments. The $1 \times 10^6$ /ml density was better than the higher density.

*Cell viability assay on exposure to A*d5bIFN$\alpha$

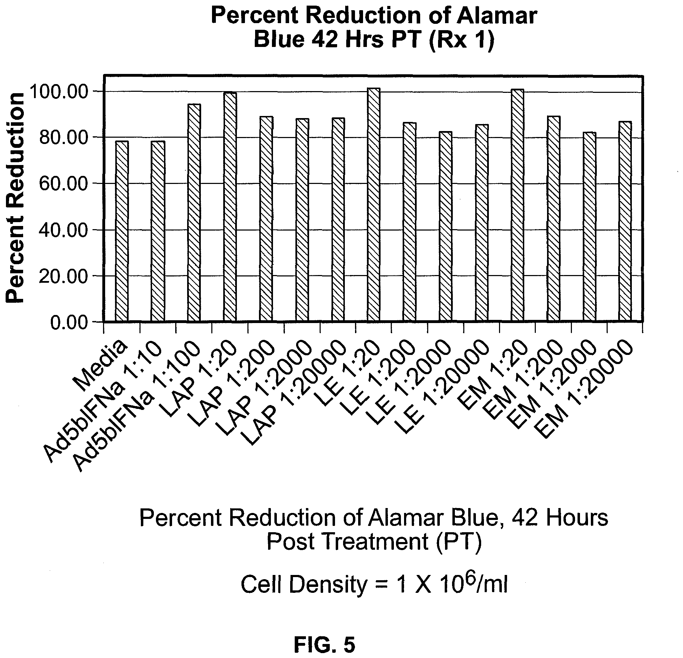

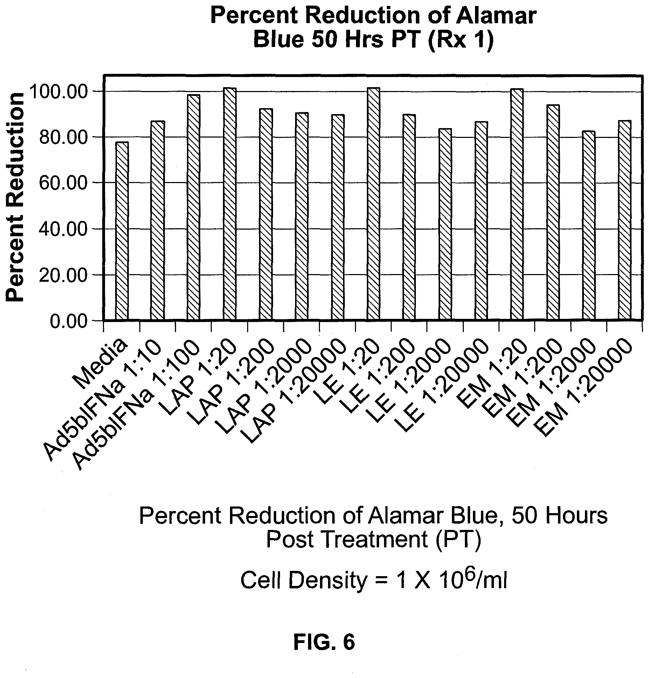

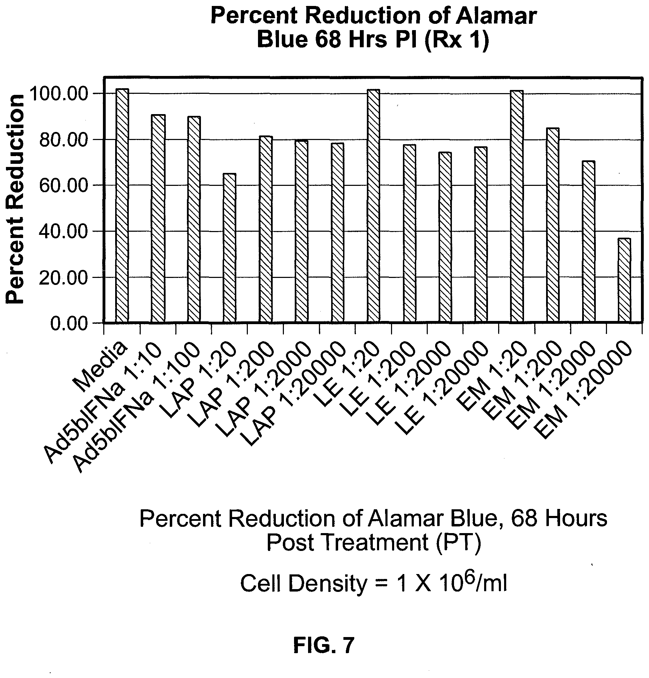

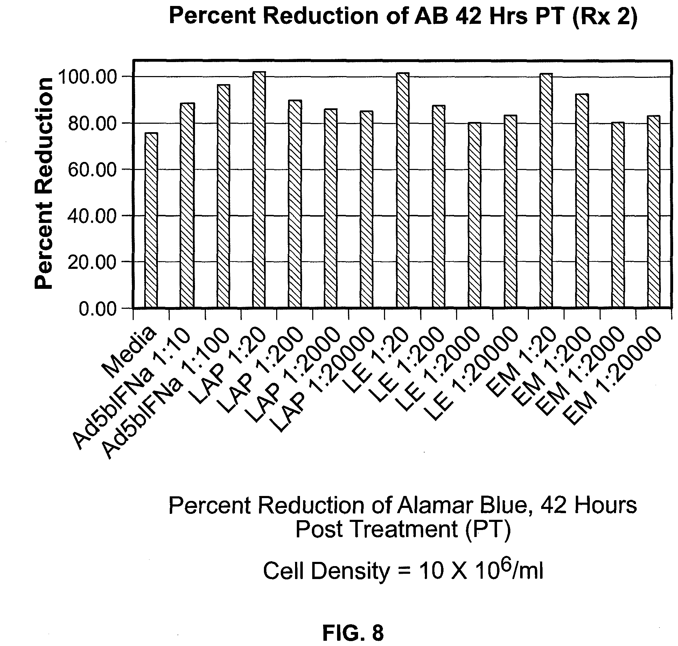

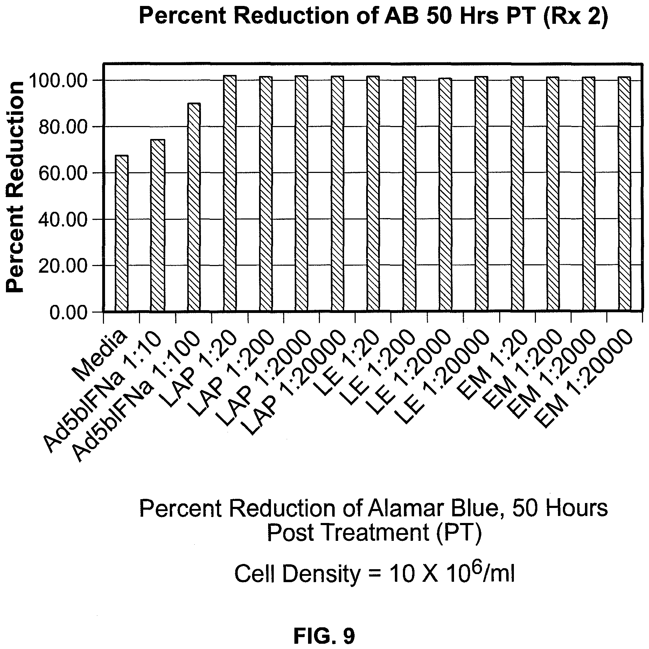

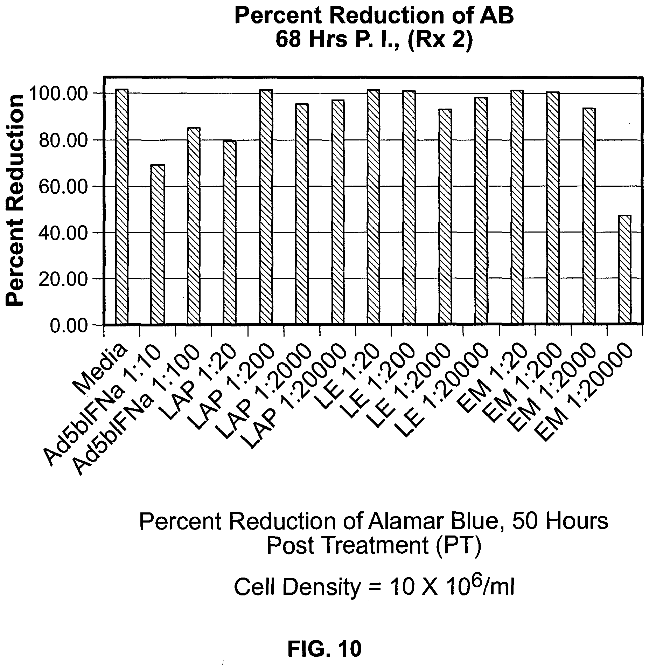

At a cell concentration of $1 \times 10^6$ /ml, the 1:100 dilution of Ad5bIFN$\alpha$ produced the higher % AB reduction than the media controls at 42 and 50 hours post exposure (Fig. 5 and 6). However, there was a % AB reduction at 68 hours post exposure (Fig 7). Similar results were obtained at a cell density of $10 \times 10^6$ /ml (Figs 8-10).

*Cell viability assay on exposure to Adjuplex LAP*

Cell exposure at a cell density of $1 \times 10^6$ /ml to *Adjuplex* LAP at a dilution of 1:20 gave the higher % AB reduction than the media controls at 42 and 50 hours post exposure (Fig. 5 and 6) but with a % AB reduction at 68 hours post exposure (Fig 7). Similar results were obtained at a cell density of $10 \times 10^6$ /ml (Figs 8-10).

*Cell viability assay on exposure to Adjuplex LE*

When 293 cells at a cell density $1 \times 10^6$ /ml were treated with Adjuplex LE at a dilution of 1:20, higher % AB reductions were obtained compared to the media controls at 42 and 50 hours post exposure (Fig. 5 and 6) but with a % AB reduction at 68 hours post exposure (Fig 7). Similar results were obtained at a cell density of $10 \times 10^6$ /ml (Figs 8-10).

*Cell viability assay on exposure to Emulsigen*

Treatment of 293 cells at a cell density $1 \times 10^6$ /ml with Emulsigen at 1:20 dilution resulted in higher % AB reductions relative to the media controls at 42 and 50 hours post exposure (Fig. 5 and 6) but with lower % AB reductions at 68 hours post exposure (Fig 7). Similar results were obtained at a cell density of $10 \times 10^6$ /ml (Figs 8-10).

*Virucidal assay ($TCID_{50}$ Assay)*

Ad5bIFN$\alpha$ virus-*Adjuvant Mixtures*

*Experiment 1*

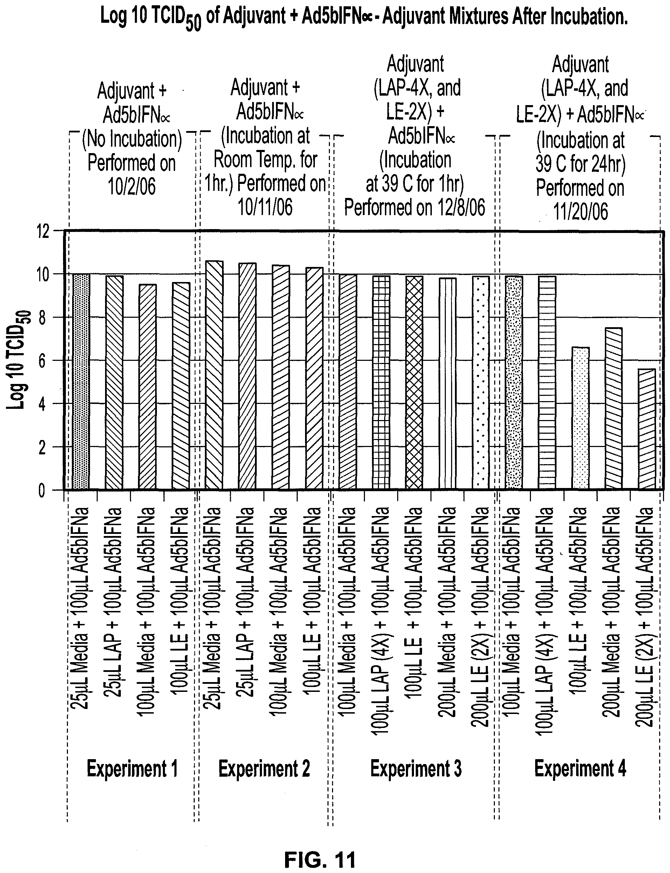

The log10 titers of treatments in this group were 10.0, 9.9, 9.5, and 9.6 (Table 11, Fig. 11). The differences between them were lower than log 0.7 (34), therefore there were no significant differences within this treatment group (34). This indicates that the two adjuvants did not produce any virucidal effect on the virus when combined at room temperature.

*Experiment 2*

As in the first experiment, leaving the virus-adjuvant mixtures at 24° C for one hour of incubation, the two adjuvants did not produce any virucidal effect on the virus because the differences between log10 titers were less than log10 0.7 (34), (Table 11, Fig. 11).

*Experiment 3*

The adjuvants in these treatment groups did not exhibit any virucidal effect on the virus when incubated for one hour at 39° C (Table 11, Fig. 11).

*Experiment 4*

Incubating the two first treatment groups at 39° C for 24 hours did not produce any virucidal effect by Adjuplex-LAP adjuvant on the virus (Table 11, Fig.11). However, treatments at these incubation time and temperature conditions significantly reduced titers by 3.3-and 1.9-log 10 $TCID_{50}$ (34) when compared to the control treatment groups at the recommended amount and when the amount of Adjuplex LE was doubled, respectively.

Ad5O1 virus-*Adjuvant Mixtures*

*Experiment 1*

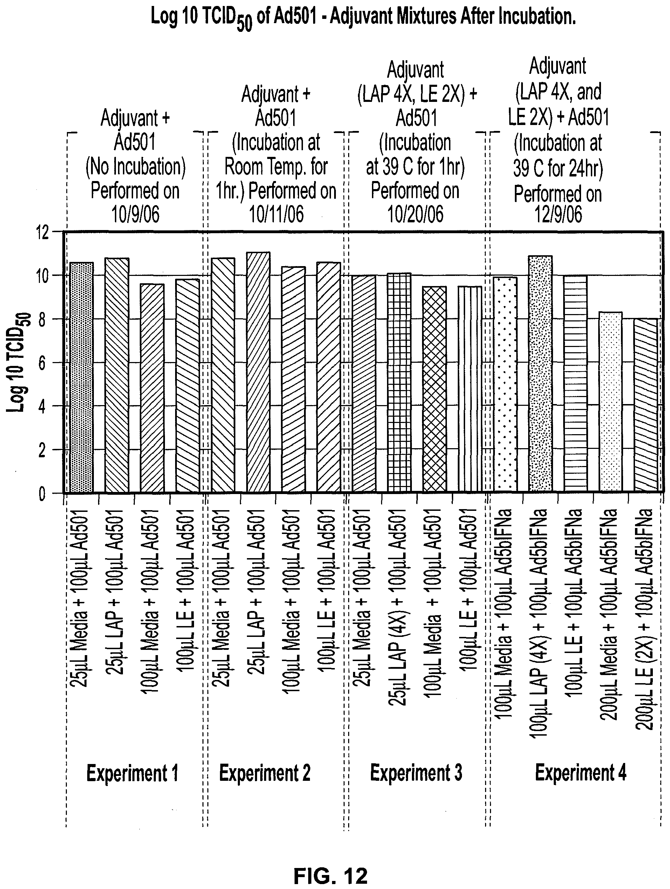

There were no significant differences in virus titers of treatments A and B. The reduction in titer between treatments C and D was 0.2 log 10 $TCID_{50}$ and this was not significant (34) (Table 12, Fig.12). Virus-adjuvant mixtures in this experiment were not incubated.

*Experiment 2*

There was no significant titer reduction between treatments A and B incubated for 1 hour at 20° C. The adjuvants did not produce any significant titer reduction (34) in treatments C and D (Table 12, Fig. 12).

*Experiment 3*

In a similar fashion, all the groups in this experiment did not show any significant differences in log10 titer reduction.

*Experiment 4*

There was a significant reduction (1.0 log 10) in titers between treatments A and B. The titer reduction (0.3 log) between treatments 3 and 4 was not significant (34) (Table 12, Fig.12).

Table 10: Alamar Blue Cytoxicity Assay Setup

| Plate 1: 1X10^5 cells per well | | | | | | | | | | | |
|---|---|---|---|---|---|---|---|---|---|---|---|
| | 1 | 2 | 3 | 4 | 5 | 6 | 7 | 8 | 9 | 10 | 11 | 12 |
| A | #1 media+cells | | | #1 Ad5-bIFNα 1:10 | | | #1 Ad5-bIFNα 1:100 | | | Media NO CELLS | | |
| B | #1 Adjuplex-LAP 1:20 | | | #1 Adjuplex-LAP 1:200 | | | #1 Adjuplex-LAP 1:2000 | | | #1 Adjuplex-LAP 1:20000 | | |
| C | #1 Adjuplex-LE 1:20 | | | #1 Adjuplex-LE 1:200 | | | #1 Adjuplex-LE 1:2000 | | | #1 Adjuplex-LE 1:20000 | | |
| D | #2 media+cells | | | #1 Ad5-bIFNα 1:10 | | | #1 Ad5-bIFNα 1:100 | | | Media NO CELLS | | |
| E | #2 Adjucplex-LAP 1:20 | | | #2 Adiuplex-LAP 1:200 | | | #2 Adjuplex-LAP 1:2000 | | | #2 Adjuplex-LAP 1:20000 | | |
| F | #2 Adjuplex-LE 1:20 | | | #2 Adjuplex-LE 1:200 | | | #2 Adjuplex-LE 1:2000 | | | #1 Adjuplex-LE 1:20000 | | |
| G | #2 Adjucplex-LAP 1:20 | | | #2 Adjuplex-LAP 1:200 | | | #2 Adiuplex-LAP 1:2000 | | | #2 Adjuplex-LAP 1:20000 | | |
| H | #2 Adiuplex-LE 1:20 | | | #2 Adjuplex-LE 1:200 | | | #2 Adjuplex-LE 1:2000 | | | #1 Adiuplex-LE 1:20000 | | |

Plate 2: 1×10^5 cells per well

| | 1 | 2 | 3 | 4 | 5 | 6 | 7 | 8 | 9 | 10 | 11 | 12 |
|---|---|---|---|---|---|---|---|---|---|---|---|---|
| A | #1 media+cells | | | #1 Ad5-bIFNα 1:10 | | | #1 Ad5-bIFNα 1:100 | | | Media NO CELLS | | |
| B | #1 Adjuplex-LAP 1:20 | | | #1 Adjuplex-LAP 1:200 | | | #1 Adjuplex-LAP 1:2000 | | | #1 Adjuplex-LAP 1:20000 | | |
| C | #1 Adjuplex-LE 1:20 | | | #1 Adjuplex-LE 1:200 | | | #1 Adjuplex-LE 1:2000 | | | #1 Adiuplex-LE 1:20000 | | |
| D | #2 media+cells | | | #1 Ad5-bIFNα 1:10 | | | #1 Ad5-bIFNα 1:100 | | | Media NO CELLS | | |
| E | #2 Adiucplex-LAP 1:20 | | | #2 Adjuplex-LAP 1:200 | | | #2 Adjuplex-LAP 1:2000 | | | #2 Adjuplex-LAP 1:20000 | | |

(continued)

|  | 1 | 2 | 3 | 4 | 5 | 6 | 7 | 8 | 9 | 10 | 11 | 12 |
|---|---|---|---|---|---|---|---|---|---|---|---|---|
| F | #2 Adiuplex-LE 1:20 | | | #2 Adjuplex-LE 1:200 | | | #2 Adjuplex-LE 1:2000 | | | #1 Adjuplex-LE 1:20000 | | |
| G | #2 Adjucplex-LAP 1:20 | | | #2 Adjuplex-LAP 1:200 | | | #2 Adjuplex-LAP 1:2000 | | | #2 Adjuplex-LAP 1:20000 | | |

**Table 11:** Ad5bIFN$\alpha$ virus-Adjuvant Mixture Titers Expressed in Log10 $TCID_{50}$

| Experiment Number | Treatment | *Temperature* | Incubation $^{0}C$ Time (hrs) | Log10 $TCID_{50}$ |
|---|---|---|---|---|
| 1 | **A.** 25 $\mu$l Media + 100$\mu$l Ad5bIFN$\alpha$ | | None | 10.0 |
| | **B.** 25 $\mu$l LAP + 100$\mu$l Ad5bIFN$\alpha$ | | None | 9.9 |
| | **C.** 100 $\mu$l LE + 100$\mu$l Ad5bIFN$\alpha$ | | None | 9.5 |
| | **D.** 100 $\mu$l Media + 100$\mu$l Ad5bIFN$\alpha$ | | None | 9.6 |
| 2 | **A.** 25 $\mu$l Media + 100$\mu$l Ad5bIFN$\alpha$ | 20 | 1 | 10.6 |
| | **B.** 25 $\mu$l LAP + 100$\mu$l Ad5bIFN$\alpha$ | 20 | 1 | 10.5 |
| | **C.** 100$\mu$l Media + 100$\mu$l Ad5bIFN$\alpha$ | 20 | 1 | 10.4 |
| | **D.** 100$\mu$l LE + 100$\mu$l Ad5bIFN$\alpha$ | 20 | 1 | 10.3 |
| 3 | **A.** 100 $\mu$l Media + 100$\mu$l Ad5bIFN$\alpha$ | 39 | 1 | 10.0 |
| | **B.** 100 $\mu$l LAP **(4X)**\* + 100$\mu$l Ad5bIFN$\alpha$ | 39 | 1 | 9.9 |
| | **C.** 100 $\mu$l LE + 100$\mu$l Ad5bIFN$\alpha$ | 39 | 1 | 9.9 |
| | **D.** 200 $\mu$l Media + 100$\mu$l Ad5bIFN$\alpha$ | 39 | 1 | 9.8 |
| | **E.** 200 $\mu$l LE **(2X)**\*\* + 100 $\mu$l Ad5bIFN$\alpha$ | 39 | 1 | 9.9 |
| 4 | **A.** 100 $\mu$l Media + 100 $\mu$l Ad5bIFN$\alpha$ | 39 | 24 | 9.9 |
| | **B.** 100 $\mu$l LAP **(4X)**\* + 100 $\mu$l Ad5bIFN$\alpha$ | 39 | 24 | 9.9 |
| | **3.** 100 $\mu$l LE + 100$\mu$l Ad5bIFN$\alpha$ | 39 | 24 | 6.6 |
| | **4.** 200 $\mu$l Media + 100 $\mu$l Ad5bIFN$\alpha$ | 39 | 24 | 7.5 |
| | **5.** 200 $\mu$l LE **(2X)**\*\* + 100 $\mu$l Ad5bIFN$\alpha$ | 39 | 24 | 5.6 |

Ad5bIFN$\alpha$ virus (without adjuvants) titer expressed in Log10 $TCID_{50}$ = 10.5

\* = Indicates four-fold in the amount of recommended concentration

\*\* = Indicates twice the amount of recommended concentration

**Table 12:** Ad5O1 virus-Adjuvant Mixture Titers Expressed in Log10 $TCID_{50}$

| Experiment Number | Treatment | *Temperature* | Incubation $^{0}C$ Time (hrs) | Log10 $TCID_{50}$ |
|---|---|---|---|---|
| 1 | **A.** 25 $\mu$l Media + 100$\mu$l Ad5O1 | | None | 10.6 |
| | **B.** 25 $\mu$l LAP + 100$\mu$l Ad5O1 | | None | 10.8 |
| | **C.** 100 $\mu$l Media + 100$\mu$l Ad5O1 | | None | 9.6 |
| | **D.** 100 $\mu$l LE + 100$\mu$l Ad5O1 | | None | 9.8 |
| 2 | **A.** 25 $\mu$l Media + 100$\mu$l Ad5O1 | 20 | 1 | 10.8 |

(continued)

| Experiment Number | Treatment | *Temperature* | Incubation $^0C$ *Time (hrs)* | Log10 TCID$_{50}$ |
|---|---|---|---|---|
| | **B.** 25 µl LAP + 100µl Ad5O1 | 20 | 1 | 11.1 |
| | **C.** 100µl Media + 100µl Ad5O1 | 20 | 1 | 10.4 |
| | **D.** 100µl LE + 100µl Ad5O1 | 20 | 1 | 10.6 |
| 3 | **A.** 100 µl Media + 100µl Ad5O1 | 39 | 1 | 10.0 |
| | **B.** 100 µl LAP **(4X)*** + 100µl Ad5O1 | 39 | 1 | 10.1 |
| | C. 200µl Media + 100µl Ad5O1 | 39 | 1 | 9.5 |
| | **D.** 200µl LE **(2X)**** + 100µl Ad5O1 | 39 | 1 | 9.5 |
| 4 | **A.** 100 µl Media + 100 µl Ad5O1 | 39 | 24 | 9.9 |
| | **B.** 100 µl LAP **(4X)*** + 100µl Ad5O1 | 39 | 24 | 10.9 |
| | **C.** 100 µl LE + 100µl Ad5O1 | 39 | 24 | 10.0 |
| | **D.** 200 µl Media + 100 µl Ad5O1 | 39 | 24 | 8.3 |
| | **E.** 200 µl LE **(2X)**** + 100 µl Ad5O1 | 39 | 24 | 8.0 |

Ad5O1 virus (without adjuvants) titer expressed in Log10 TCID$_{50}$ = 11.8

* = Indicates four-fold in the amount of recommended concentration

** = Indicates twice the amount of recommended concentration

DISCUSSION

Alamar Blue is a redox indicator of viable cell number. At cell density of $10 \times 10^6$/ml, cell clumping was observed, which indicated that there were too many cells in each well at this concentration. There was no clumping at a cell concentration of $1 \times 10^6$/ml. Based on this observation, the cell concentration $1 \times 10^6$/ml was employed in the study. In this study AB was employed to measure 293 cell viability at two cell concentrations. Optical densities were measured at 570 nm and 600 nm at approximately 42 hours (total culture time), 50 hours, and 68 hours. The OD data obtained were analyzed using the formula provided by the AB manufacturer. The % difference in reduction of AB (between media growth control wells and treatment wells) indicated the amount inhibition (or stimulation) of cell growth in treatment wells with respect to media control wells.

Treatments with higher % reductions than media controls are considered to stimulate cell growth. Treatments with lower % reduction than media controls are considered cytotoxic. Treatments with the same % reduction are neither cytotoxic nor stimulatory.

A dilution of 1:100 of Ad5bIFN$\alpha$ virus gave the higher % reduction of AB at 42 and 50 hours post exposure to the 293 cells at both $1 \times 10^6$/ml and $10 \times 10^6$/ml. The $1 \times 10^6$/ml cell concentration would be the best concentration because there was no cell clumping observed at a cell concentration of $1 \times 10^6$/ml. The 1:100 dilution would be the optimal dilution of Ad5bIFN$\alpha$ virus for 293 cells. Adjuplex LAP, LE, and emulsigen adjuvants at dilutions of 1:20 gave the highest percent reduction of AB at 42 and 50 hours post exposure to 293 cells.

When LAP and LE were mixed at room temperature with Ad5bIFN$\alpha$ virus and immediately assayed for virus titer, there was no significant reduction in log titer. A similar result was obtained when the virus-adjuvant mixtures were incubated for one hour.

There was no significant virus titer reduction in the virus-adjuvant mixtures when incubated for one hour at room temperature. The adjuvant contents of LAP and LE of the mixtures were increased four-fold and two fold, respectively, in order to increase the possibility of these adjuvants having a virucidal effect on the viruses. However, the reduction in titer was very little. When the incubation temperature was raised to $39^0$ C but incubation time remaining the same, there was no appreciable reduction in titer.

The virus-adjuvant mixtures were incubated with 4- and 2-fold in the LAP and LE contents, respectively, for 24 hours (previously it was one hour), there was no reduction in titer of the virus-adjuvant LAP mixture. However, there was a 1.9 log 10 reduction in titer of the virus-LE mixture. This reduction was significant (35). The titer reduction 3.3 log 10 was even greater when the amount of LE in the virus-adjuvant mixture was not increased.

The conclusion drawn from this study was that adjuplex LAP did not have any virucidal effect on Ad5bIFN$\alpha$ virus at the recommended amount and even when the amount was increased by 4. The incubation temperatures and times for this observation were 20° C, 39⁰ C, 1 and 24 hours. However, the same could not be said for adjuplex LE because although there were no titer reductions when the incubation temperature and time were 39° C and one hour, when the time of incubation was increased to 24 hours there were significant reductions in virus titers at both concentrations. Results from this study would suggest that Adjuplex-LE adjuvant produced a virucidal effect on Ad5blIFN$\alpha$ virus when the adjuvant concentration in the mixture was increased two-fold., and also at the recommended concentration.

Similar results were obtained for Ad5O1 virus was mixed with both adjuplex LAP and LE at the recommended quantity and when the amount was increased by 4 fold and 2 fold, respectively. Furthermore, there were no significant titer reductions even when the mixtures were incubated at 39⁰ C for 24 hours.

The 39⁰ C incubation temperature was selected because that is the average rectal temperature of cattle. It is recommended that adjuplex LAP should be the adjuvant to be combined with the Ad5blIFN$\alpha$, Ad5O1 and other Ad5 based FMD sub unit vaccines for clinical evaluation in cattle.

EXAMPLE 13

Vaccination of Chickens with H5 HA-Transfected Cells and its Effect on Detectable Shedding of Low Pathogenic Avian Influenza Virus Following Challenge by Real-time Reverse Transcriptase Polymerase Chain Reaction

OBJECTIVE

The objective of this study was to challenge chickens vaccinated with H5N2 low pathogenic avian influenza (LPAI) virus and evaluate effect by measuring virus shed from the oropharynx and the cloaca by real-time reverse transcriptase polymerase chain reaction.

BACKGROUND

A further purpose was to evaluate if Chinese Hamster Ovary (CHO) cells, stably transfected with H5 HA expressing plasmid, would stimulate an immune response in birds. Following one vaccination, seroconversion to H5 HA was noted in a few birds. The birds were subsequently administered a second dose. Since the birds seroconverted following one dose, the birds were challenged with H5N2 LPAI virus to determine if the detectable serological response had any effect on virus shed.

TEST / CONTROL ARTICLES

| | | |
|---|---|---|
| 1. | Generic Name: | Media / Lecithin acrylic copolymer plus Quil A cholesterol |
| | Formulation: | Media - DMEM, fetal calf serum, non-essential amino acids, L-glutamine |
| 2. | Generic Name: | Control Cells / Lecithin acrylic copolymer plus Quil A cholesterol |
| | Formulation: | Control cells - CHO cells not expressing HA |
| 3. | Generic Name: | CHO-HA-10 cells freeze/thaw / Lecithin acrylic copolymer plus Quil A cholesterol |
| | Formulation: | CHO-HA-10 - CHO cells transfected to express HA with a freeze/thaw application |
| 4. | Generic Name: | CHO-HA-10 cells fresh / Lecithin acrylic copolymer plus Quil A cholesterol |
| | Formulation: | CHO-HA-10 - CHO cells transfected to express HA prepared fresh |

CHALLENGE ORGANISM

| | |
|---|---|
| Description: | LPAI* virus isolate A/TK/CA/209092/02 (H5N2) |
| Origin: | National Veterinary Services Laboratory |
| Dosage: | Challenge dose of $10^{5.5}$ ELD$_{50}$** per 0.1 ml dose |
| Route of Infection: | Intranasal |

*LPAI - Low pathogenic avian influenza
**ELD$_{50}$ - Embryo lethal dose 50

Table 13: STUDY ANIMALS

| Species: | Chickens | Type: | SPF |
|---|---|---|---|
| Breed / Strain: | Leghorn | Sex: | Male and Female |
| Description: | Individually identified | Age: | 64 days at challenge |
| Origin (chicks) | Hy-Vac 21459 Old Hwy 6 Adel, Iowa 50003 | Total: | 15 |
| Birds were transferred from IACUC request BEDA 1197-06-06 for challenge. | | | |

Table 14: STUDY DESIGN

| Trt. No. | Treatment Group | | Bird Numbers | LPAI IN* Challenge Day 0 | Sample** Days |
|---|---|---|---|---|---|
| | Antigen | Adjuvant | | | |
| T1 | None | None | 3 | H5N2 $10^{5.5}$ ELD$_{50}$# | 0 through 6 |
| T2 | Media## | LAP/QAC† | 3 | H5N2 $10^{5.5}$ ELD$_{50}$# | 0 through 6 |
| T3 | Control cells†† | LAP/QAC† | 3 | H5N2 $10^{5.5}$ ELD$_{50}$# | 0 through 6 |
| T4 | CHO-HA-10 cells‡ freeze/thaw | LAP/QAC† | 3 | H5N2 $10^{5.5}$ ELD$_{50}$# | 0 through 6 |
| T5 | CHO-HA-10 cells‡ fresh | LAP/QAC† | 3 | H5N2 $10^{5.5}$ ELD$_{50}$# | 0 through 6 |

*LPAI IN - Low pathogenic avian influenza virus isolate A/TK/CA/209092/02 (H5N2), intranasal administration with 0.1 ml.

**Sample Days - Oropharyngeal and cloacal samples collected. Samples were evaluated by real-time reverse transcriptase polymerase chain reaction. On Day 0, blood samples were also collected that were evaluated by hemagglutination inhibition.

#H5N2 $10^{5.5}$ ELD$_{50}$ - Low pathogenic avian influenza isolate A/TK/CA/209092/02 administered intranasally at a dose of $10^{5.5}$ embryo lethal dose 50 per 0.1 ml challenge inoculum.

##Media - DMEM, fetal calf serum, non-essential amino acids, L-glutamine.

†LAP/QAC - Lecithin acrylic copolymer plus Quil A cholesterol.

††Control cells - CHO cells not expressing HA.

‡CHO-HA-10 cells - CHO cells transfected to express HA.

PROCEDURES

Prior to Day 0

Leghorn specific-pathogen-free chicks used in the study were derived from IACUC request BEDA 1197-06-06. Birds were placed in five isolators, three birds per isolator with each isolator housing a treatment group.

Day 0

On Day 0, a blood sample, an oropharyngeal swab, and a cloacal swab were collected from each bird. Oropharyngeal and cloacal swabs were placed into one ml of culture medium and frozen at approximately -80° C until processed for viral detection.

Also on Day 0, all birds were exposed by the intranasal route to 0.1 ml of challenge inoculum of LPAI H5N2 isolate according to the table under STUDY DESIGN. The titer of the inoculum was $10^{5.5}$ ELD$_{50}$ per 0.1-ml dose.

Days 1 through 6

On Days 1 through 6, an oropharyngeal swab and a cloacal swab were collected from each bird in all treatment groups and processed as described previously.

All birds were euthanized and disposed of according to standard operating procedures following sample collection on Day 6.

Serum Testing

Serum samples collected from birds on Day 0 were analyzed for hemagglutination inhibition (HAI) titers against H5 avian influenza virus (A/Turkey/Wisconsin/68 [H5N9]). Results of serological testing on serum samples collected from birds while on the BEDA 1197-06-06 IACUC Request Study have been incorporated into this report.

Virus Detection

Oropharyngeal swabs collected on Days 0 through 6 and cloacal swabs collected on Days 2 through 6 from birds in treatment groups T1, T3, T4, and T5 were analyzed for viral RNA by RT-rtPCR. Samples collected from birds in treatment group T2 were not analyzed and cloacal samples collected on Days 0 and 1 from the remaining treatment groups were also not analyzed. RNA extraction for the RT-rtPCR assay was conducted in the laboratory at 2321 30 Road, Brainard, Nebraska. The RT-rtPCR assay was conducted in the laboratory at 521 West Industrial Lake Drive, Lincoln, Nebraska.

Deviations to the Protocol

According to the Benchmark Biolabs IACUC proposal (BEDA 1197-06-06) under the direction of standard operating procedure AC-019-01, first vaccination was to take place when birds were four to six weeks of age. Birds were approximately 26 days of age at time of first vaccination. This deviation had no impact on the study.

According to the Benchmark Biolabs IACUC proposal (BEDA 1197-06-06) under the direction of standard operating procedure AC-019-01, the media used in treatment group 2 (T2) was to contain fetal calf serum. However, due to the cost of serum and the fact that the cells used for inoculation in T3, T4, and T5 groups were rinsed, serum was not added as it was determined that the addition or elimination of serum would not effect the antibody response to the test antigen in this study. Therefore, this deviation had no impact on the study.

Leghorn specific-pathogen-free chicks were used in the study and were derived from IACUC request BEDA 1197-06-06. Birds were to be placed in five isolators, three birds per isolator; however, birds were placed into individual isolators by treatment group rather than by placement of birds from different treatment groups in each isolator. In addition, the documentation for placement into the isolators was not done. This deviation impacted the study in that there was no effort to control for isolator effect on individual treatment groups which could have had undetected consequences on one or more treatment groups.

DATA ANALYSIS

Descriptive statistics were conducted on data collected.

RESULTS AND DISCUSSION

> **Table 15.** Avian influenza hemagglutination inhibition (HAI) assay titers
> **Table 16.** Geometric mean titers for real-time reverse transcriptase polymerase chain reaction assay results on oropharyngeal swab samples
> **Table 17.** Geometric mean titers for real-time reverse transcriptase polymerase chain reaction assay results on cloacal swab samples
> **Table 18.** Real-time reverse transcriptase polymerase chain reaction assay results on oro-pharyngeal swab samples
> **Table 19.** Real-time reverse transcriptase polymerase chain reaction assay results on cloacal swab samples

Table 15 lists the results of the avian influenza H5 HAI serological testing. Following first vaccination on Day -25 on the BEDA 1197-06-06 IACUC Request Study, one of three birds in treatment group T4 and two of three birds in treatment group T5 had detectable H5 serological titers of either 8 or 16. On Day 0, all birds in treatment groups T4 and T5 had seroconverted to H5 with titers ranging from 8 to 128. No birds in treatment groups T1 through T3 had detectable H5 serological titers on either sampling day. These detectable titers were considered substantial due to the fact that the CHO cells were transfected with H5 (A/Chicken/Scotland 59 [HSN1]) that was heterologous to the H5 antigen in the serological assay (A/Turkey/Wisconsin 68 [H5N9]).

Table 16 lists the geometric mean titer (GMT) results of the oropharyngeal swab testing. No viral RNA was detected on Day 0 from any treatment groups. For Days 1 through 6, virus levels were detected by RT-rtPCR in all three T1 birds (negative controls) with the peak mean titer occurring on Day 1 (GMT of $7.00 \times 10^7$ viral copy number) and a secondary peak titer occurring on Day 4 (GMT of $3.37 \times 10^7$ viral copy number). The viral copy number of the three T1 birds declined to a GMT of $7.55 \times 10^4$ by Day 6. In birds that were administered only non-transfected CHO cells (T3) or CHO cells transfected to express H5 HA (T4 and T5), similar levels of viral copy number were detected on Days 1 through 6 when compared to treatment group T1. This indicates that vaccination with either fresh H5-transfected CHO cells or frozen and thawed H5-transfected CHO cells had no effect on A/TK/CA/209092/02 (H5N2) oropharyngeal viral shedding.

Table 17 lists the geometric mean titer (GMT) results of the cloacal swab testing. For Days 2 through 6, virus levels were detected by RT-rtPCR in all three T1 birds (negative controls) with the peak mean titer occurring on Day 5 (GMT of $4.78 \times 10^6$ viral copy number). In general, the shedding was more variable than that detected in oropharyngeal swabs from the same birds. In birds that were administered only non-transfected CHO cells (T3) or CHO cells transfected to express H5 HA (T4 and T5), generally lower levels of viral copy number were detected on Days 2 through 6 when compared to treatment group T1; however, the results varied greatly from bird to bird and day to day. This suggests that measuring vaccine effects on fecal shedding via cloacal swabs may be difficult with A/TK/CA/209092/02 (H5N2) and the effect of vaccination in this trial could not be evaluated.

CONCLUSION

It is concluded that vaccination with fresh H5-transfected CHO cells or frozen and thawed H5-transfected CHO cells induced detectable titers in a heterologous H5 antigen serological assay but had no effect on A/TK/CA/209092/02 (H5N2) oropharyngeal viral shedding. Fecal shedding of A/TK/CA/209092/02 (H5N2) could not be evaluated due to highly variable shedding detected in cloacal swabs.

It is also concluded that Quil A and cholesterol when used in combination with other adjuvant based embodiments described herein have surprising utility in the context of the present invention.

Table 15. Avian influenza hemagglutination inhibition (HAI) assay titers

| Trt No. | Treatment Group | | Bird Number | H5 HAI titer Day -25* | H5 HAI titer Day 0** |
|---|---|---|---|---|---|
| | Antigen | Adjuvant | | | |
| T1 | None | None | 67 | <8 | <2 |
| | | | 68 | <8 | <2 |
| | | | 69 | <8 | <2 |
| T2 | Media# | LAP/QAC## | 70 | <8 | <2 |
| | | | 71 | <8 | <2 |
| | | | 72 | <8 | <2 |
| T3 | Control | LAP/QAC## | 73 | <8 | <2 |
| | | | 74 | <8 | <2 |
| | | | 75 | <8 | <2 |
| T4 | CHO-HA-10 cells†† freeze/thaw | LAP/QAC## | 76 | 8 | 64 |
| | | | 77 | <8 | 64 |
| | | | 78 | <8 | 128 |

(continued)

| Trt No. | Treatment Group | | Bird Number | H5 HAI titer Day -25* | H5 HAI titer Day 0** |
|---|---|---|---|---|---|
| | Antigen | Adjuvant | | | |
| T5 | CHO-HA-10 cells[††] fresh | LAP/QAC[##] | 79 | <8 | 8 |
| | | | 80 | 8 | 128 |
| | | | 81 | 16 | 128 |

*H5 HAI titer Day -25 - Hemagglutination inhibition titer to H5N9 avian influenza virus.
**H5 HAI titer Day 0 - Hemagglutination inhibition titer to H5N9 avian influenza virus.
[#]Media - DMEM, non-essential amino acids, L-glutamine.
[##]LAP/QAC - Lecithin acrylic copolymer plus Quil A cholesterol.
[†]Control cells - CHO cells not expressing HA.
[††]CHO-HA-10 cells - CHO cells transfected to express HA.

Table 16. Geometric mean titers for real-time reverse transcriptase polymerase chain reaction assay results on oropharyngeal swab samples

| Trt No. | Treatment Group | | Study Day | RT-rtPCR* | |
|---|---|---|---|---|---|
| | Antigen | Adjuvant | | Number of birds positive / total birds | GMT viral copy number** |
| T1 | None | None | 0 | 0/3 | $0.00 \times 10^0$ |
| | | | 1 | 3/3 | $7.00 \times 10^7$ |
| | | | 2 | 3/3 | $8.32 \times 10^6$ |
| | | | 3 | 3/3 | $1.40 \times 10^7$ |
| | | | 4 | 3/3 | $3.37 \times 10^7$ |
| | | | 5 | 3/3 | $6.64 \times 10^6$ |
| | | | 6 | 3/3 | $7.55 \times 10^4$ |
| T2 | Media[#] | LAP/QAC[##] | 0 | Not done | Not done |
| | | | 1 | Not done | Not done |
| | | | 2 | Not done | Not done |
| | | | 3 | Not done | Not done |
| | | | 4 | Not done | Not done |
| | | | 5 | Not done | Not done |
| | | | 6 | Not done | Not done |
| T3 | Control | LAP/QAC[##] | 0 | 0/3 | $0.00 \times 10^0$ |
| | | | 1 | 3/3 | $5.79 \times 10^6$ |
| | | | 2 | 3/3 | $2.37 \times 10^7$ |
| | | | 3 | 3/3 | $6.70 \times 10^6$ |
| | | | 4 | 3/3 | $1.15 \times 10^7$ |
| | | | 5 | 3/3 | $3.80 \times 10^6$ |
| | | | 6 | 3/3 | $1.03 \times 10^6$ |

(continued)

| Trt No. | Treatment Group | | Study Day | RT-rtPCR* | |
|---|---|---|---|---|---|
| | Antigen | Adjuvant | | Number of birds positive / total birds | GMT viral copy number** |
| T4 | CHO-HA-10 cells†† freeze/thaw | LAP/QAC## | 0 | 0/3 | $0.00 \times 10^0$ |
| | | | 1 | 3/3 | $9.33 \times 10^6$ |
| | | | 2 | 3/3 | $4.55 \times 10^6$ |
| | | | 3 | 3/3 | $6.46 \times 10^6$ |
| | | | 4 | 3/3 | $4.46 \times 10^7$ |
| | | | 5 | 3/3 | $5.91 \times 10^6$ |
| | | | 6 | 3/3 | $4.39 \times 10^6$ |
| T5 | CHO-HA-10 cells†† fresh | LAP/QAC## | 0 | 0/3 | $0.00 \times 10^0$ |
| | | | 1 | 3/3 | $9.23 \times 10^6$ |
| | | | 2 | 3/3 | $8.17 \times 10^6$ |
| | | | 3 | 3/3 | $1.04 \times 10^7$ |
| | | | 4 | 3/3 | $9.58 \times 10^7$ |
| | | | 5 | 3/3 | $7.87 \times 10^6$ |
| | | | 6 | 3/3 | $2.95 \times 10^6$ |

*RT-rtPCR - Real-time reverse transcriptase polymerase chain reaction assay.

**GMT viral copy number - Geometric mean titer viral copy number. Samples where no copies were detected were factored as 1 for calculation of GMT.

#Media - DMEM, non-essential amino acids, L-glutamine.

##LAP/QAC - Lecithin acrylic copolymer plus Quil A cholesterol.

†Control cells - CHO cells not expressing HA.

††CHO-HA-10 cells - CHO cells transfected to express HA.

Table 17. Geometric mean titers for real-time reverse transcriptase polymerase chain reaction assay results on cloacal swab samples

| Trt . No. | Treatment Group | | Study Day | RT-rtPCR* | |
|---|---|---|---|---|---|
| | Antigen | Adjuvant | | Number of birds positive / total birds | GMT viral copy number** |
| | | | 0 | Not done | Not done |
| T1 | None | None | 1 | Not done | Not done |
| | | | 2 | 3/3 | $5.46 \times 10^5$ |
| | | | 3 | 3/3 | $1.34 \times 10^5$ |
| | | | 4 | 3/3 | $5.66 \times 10^5$ |
| | | | 5 | 3/3 | $4.78 \times 10^6$ |
| | | | 6 | 3/3 | $2.03 \times 10^6$ |

(continued)

| Trt . No. | Treatment Group | | Study Day | RT-rtPCR* | |
|---|---|---|---|---|---|
| | Antigen | Adjuvant | | Number of birds positive / total birds | GMT viral copy number** |
| T2 | Media# | LAP/QAC## | 0 | Not done | Not done |
| | | | 1 | Not done | Not done |
| | | | 2 | Not done | Not done |
| | | | 3 | Not done | Not done |
| | | | 4 | Not done | Not done |
| | | | 5 | Not done | Not done |
| | | | 6 | Not done | Not done |
| T3 | Control | LAP/QAC## | 0 | Not done | Not done |
| | | | 1 | Not done | Not done |
| | | | 2 | 1/3 | $3.64 \times 10^1$ |
| | | | 3 | 2/3 | $4.90 \times 10^2$ |
| | | | 4 | 3/3 | $1.13 \times 10^5$ |
| | | | 5 | 3/3 | $3.35 \times 10^5$ |
| | | | 6 | 2/3 | $6.86 \times 10^4$ |
| T4 | CHO-HA-10 cells†† freeze/thaw | LAP/QAC## | 0 | Not done | Not done |
| | | | 1 | Not done | Not done |
| | | | 2 | 2/3 | $1.05 \times 10^3$ |
| | | | 3 | 2/3 | $1.06 \times 10^4$ |
| | | | 4 | 3/3 | $8.55 \times 10^5$ |
| | | | 5 | 3/3 | $1.12 \times 10^6$ |
| | | | 6 | 1/3 | $1.95 \times 10^2$ |
| T5 | CHO-HA-10 cells†† fresh | LAP/QAC## | 0 | Not done | Not done |
| | | | 1 | Not done | Not done |
| | | | 2 | 3/3 | $3.31 \times 10^4$ |
| | | | 3 | 3/3 | $5.15 \times 10^4$ |
| | | | 4 | 3/3 | $7.19 \times 10^5$ |
| | | | 5 | 3/3 | $1.39 \times 10^5$ |
| | | | 6 | 3/3 | $1.01 \times 10^6$ |

*RT-rtPCR - Real-time reverse transcriptase polymerase chain reaction assay.

**GMT viral copy number - Geometric mean titer viral copy number. Samples where no copies were detected were factored as 1 for calculation of GMT.

#Media - DMEM, non-essential amino acids, L-glutamine.

##LAP/QAC - Lecithin acrylic copolymer plus Quil A cholesterol.

†Control cells - CHO cells not expressing HA.

††CHO-HA-10 cells - CHO cells transfected to express HA.

Table 18. Real-time reverse transcriptase polymerase chain reaction assay results on oropharyngeal swab samples

| Trt. Group | Bird number | RT-rtPCR* (Mean viral copy number)** on oropharyngeal swabs | | | | | | |
|---|---|---|---|---|---|---|---|---|
| | | Day 0 | Day 1 | Day 2 | Day 3 | Day 4 | Day 5 | Day 6 |
| T1 Antigen - none Adjuvant - none | 67 | $0.00 \times 10^0$ | $2.07 \times 10^8$ | $7.87 \times 10^6$ | $1.67 \times 10^7$ | $2.54 \times 10^7$ | $6.48 \times 10^6$ | $1.91 \times 10^5$ |
| | 68 | $0.00 \times 10^0$ | $3.70 \times 10^7$ | $7.94 \times 10^6$ | $7.03 \times 10^7$ | $2.00 \times 10^7$ | $3.48 \times 10^6$ | $2.38 \times 10^4$ |
| | 69 | $0.00 \times 10^0$ | $4.48 \times 10^7$ | $9.22 \times 10^6$ | $2.36 \times 10^6$ | $7.56 \times 10^7$ | $1.30 \times 10^7$ | $9.47 \times 10^4$ |
| T2 Media# LAP/QAC## | 70 | Not done | Not done | Not done | Not done | Not done | Not done | Not done |
| | 71 | Not done | Not done | Not done | Not done | Not done | Not done | Not done |
| | 72 | Not done | Not done | Not done | Not done | Not done | Not done | Not done |
| T3 Control LAP/QAC## | 73 | $0.00 \times 10^0$ | $1.20 \times 10^7$ | $7.44 \times 10^5$ | $1.63 \times 10^7$ | $1.70 \times 10^7$ | $2.19 \times 10^6$ | $4.03 \times 10^\circ$ |
| | 74 | $0.00 \times 10^0$ | $9.39 \times 10^6$ | $4.92 \times 10^6$ | $1.25 \times 10^7$ | $5.66 \times 10^6$ | $1.10 \times 10^7$ | $1.40 \times 10^6$ |
| | 75 | $0.00 \times 10^0$ | $1.72 \times 10^6$ | $3.63 \times 10^9$ | $1.48 \times 10^6$ | $1.59 \times 10^7$ | $2.26 \times 10^6$ | $1.92 \times 10^5$ |
| T4 CHO-HA-10 cells†† freeze thaw LAP/QAC## | 76 | $0.00 \times 10^0$ | $3.04 \times 10^7$ | $5.57 \times 10^6$ | $1.50 \times 10^7$ | $6.04 \times 10^7$ | $2.86 \times 10^6$ | $1.43 \times 10^6$ |
| | 77 | $0.00 \times 10^0$ | $1.87 \times 10^7$ | $3.04 \times 10^6$ | $3.86 \times 10^6$ | $6.16 \times 10^7$ | $6.67 \times 10^6$ | $1.05 \times 10^7$ |
| | 78 | $0.00 \times 10^0$ | $1.43 \times 10^6$ | $5.55 \times 10^6$ | $4.65 \times 10^6$ | $2.38 \times 10^7$ | $1.08 \times 10^7$ | $5.63 \times 10^6$ |
| T5 CHO-HA-10 cell†† fresh LAP/QAC## | 79 | $0.00 \times 10^0$ | $4.41 \times 10^6$ | $7.06 \times 10^7$ | $3.05 \times 10^6$ | $1.61 \times 10^7$ | $1.07 \times 10^7$ | $4.65 \times 10^6$ |
| | 80 | $0.00 \times 10^0$ | $1.51 \times 10^7$ | $5.32 \times 10^6$ | $1.07 \times 10^7$ | $1.23 \times 10^8$ | $1.86 \times 10^7$ | $4.11 \times 10^6$ |
| | 81 | $0.00 \times 10^0$ | $1.18 \times 10^7$ | $1.45 \times 10^6$ | $3.42 \times 10^7$ | $4.43 \times 10^8$ | $2.45 \times 10^6$ | $1.34 \times 10^6$ |

*RT-rtPCR - Real-time reverse transcriptase polymerase chain reaction assay.

**Mean titer viral copy number - Samples where no copies were detected were factored as 0 for calculation of means.

#Media - DMEM, non-essential amino acids, L-glutamine.

##LAP/QAC - Lecithin acrylic copolymer plus Quil A cholesterol.

†Control cells - CHO cells not expressing HA.

††CHO-HA-10 cells - CHO cells transfected to express HA

Table 19. Real-time reverse transcriptase polymerase chain reaction assay results on cloacal swab samples

| Trt. Group | Bird number | RT-rtPCR* (Mean viral copy number)** on cloacal swabs | | | | | | |
|---|---|---|---|---|---|---|---|---|
| | | Day 0 | Day 1 | Day 2 | Day 3 | Day 4 | Day 5 | Day 6 |
| T1 Antigen - none Adjuvant - none | 67 | Not done | Not done | $2.81 \times 10^8$ | $1.29 \times 10^6$ | $4.42 \times 10^7$ | $6.58 \times 10^8$ | $4.99 \times 10^7$ |
| | 68 | Not done | Not done | $4.34 \times 10^4$ | $5.51 \times 10^4$ | $6.07 \times 10^4$ | $3.94 \times 10^4$ | $1.37 \times 10^4$ |
| | 69 | Not done | Not done | $1.34 \times 10^4$ | $3.41 \times 10^4$ | $6.74 \times 10^4$ | $4.21 \times 10^6$ | $1.23 \times 10^7$ |
| T2 Media# LAP/QAC## | 70 | Not done | Not done | Not done | Not done | Not done | Not done | Not done |
| | 71 | Not done | Not done | Not done | Not done | Not done | Not done | Not done |
| | 72 | Not done | Not done | Not done | Not done | Not done | Not done | Not done |
| T3 Control cells† LAP/QAC## | 73 | Not done | Not done | $4.81 \times 10^4$ | $1.48 \times 10^4$ | $8.33 \times 10^4$ | $1.89 \times 10^4$ | $0.00 \times 10^0$ |
| | 74 | Not done | Not done | $0.00 \times 10^0$ | $0.00 \times 10^0$ | $1.75 \times 10^5$ | $8.54 \times 10^6$ | $5.43 \times 10^7$ |
| | 75 | Not done | Not done | $0.00 \times 10^0$ | $7.96 \times 10^3$ | $9.93 \times 10^4$ | $2.33 \times 10^5$ | $5.95 \times 10^6$ |
| T4 CHO-HA-10 cells†† freeze thaw LAP/QAC## | 76 | Not done | Not done | $2.65 \times 10^4$ | $2.61 \times 10^4$ | $2.42 \times 10^5$ | $8.58 \times 10^4$ | $0.00 \times 10^0$ |
| | 77 | Not done | Not done | $0.00 \times 10^0$ | $0.00 \times 10^0$ | $4.69 \times 10^4$ | $2.46 \times 10^5$ | $0.00 \times 10^0$ |
| | 78 | Not done | Not done | $4.34 \times 10^4$ | $4.57 \times 10^7$ | $5.51 \times 10^7$ | $6.69 \times 10^7$ | $7.41 \times 10^6$ |
| T5 | 79 | Not done | Not done | $2.66 \times 10^4$ | $3.83 \times 10^5$ | $2.02 \times 10^6$ | $2.85 \times 10^6$ | $1.30 \times 10^6$ |
| CHO-HA-10 cells†† fresh LAP/QAC## | 80 | Not done | Not done | $5.84 \times 10^4$ | $1.50 \times 10^4$ | $4.23 \times 10^4$ | $3.90 \times 10^4$ | $1.08 \times 10^7$ |
| | 81 | Not done | Not done | $2.34 \times 10^4$ | $2.38 \times 10^4$ | $4.33 \times 10^6$ | $2.44 \times 10^4$ | $7.34 \times 10^4$ |

*RT-rtPCR - Real-time reverse transcriptase polymerase chain reaction assay.

**Mean titer viral copy number - Samples where no copies were detected were factored as 0 for calculation of means.

#Media - DMEM, non-essential amino acids, L-glutamine.

##LAP/QAC - Lecithin acrylic copolymer plus Quil A cholesterol.

†Control cells - CHO cells not expressing HA.

††CHO-HA-10 cells - CHO cells transfected to express HA.

EXAMPLE 14

SUMMARY

Vaccines prepared with LAP or LAP/QAC adjuvants stimulate serologic responses in poultry, mice, swine, and elicit protection from live NDV challenge in poultry; however, the mechanism of immune response development to these vaccines has not been studied. The present study was designed to investigate the cytokine profiles of splenic lymphocytes from mice after a prime and boost regimen with LAP or LAP/QAC adjuvant, with and without antigen. After in vitro stimulation, splenic lymphocytes from some animals vaccinated with LAP + OVA or LAP/QAC + OVA produced increased levels of IL-4 mRNA relative to media controls. Large variations in IL-4 mRNA expression were observed between animals in each treatments group, suggesting that further optimization of sampling times is required. Samples were also evaluated for relative expression if IFN-$\gamma$ and TNF-$\alpha$; however, increase in expression of these cytokine mRNAs were not observed.

INTRODUCTION

Cytokine responses that develop after vaccination with LAP or LAP/QAC adjuvants, alone or in combination with antigen, are not well characterized. Polarized cytokine responses, classified as T-helper 1 (Th1) or T-helper 2 (Th2), are initiated within hours of vaccination. The nature of the cytokine profile predicts whether the immune response favors cellular (Th1) or humoral (Th2) immunity. Identification of the cytokines produced after vaccination with LAP or LAP/QAC-adjuvanted vaccines can provide clues about the mechanism of immune induction. Methods developed through this study facilitate investigation into Th1 and Th2 cytokine responses associated with LAP-and LAP/QAC-adjuvanted vaccines.

**Table 1. Study Design**

| Treatment Group | Vaccine | No. Mice | Vaccination | | | Bleed / Necropsy Day |
| | | | Days | Dose | Route of Administration | Day 16 |
|---|---|---|---|---|---|---|
| T1 | None | 4 | N/A | N/A | N/A | 4 mice |
| T2 | LAP* | 4 | 0,14 | 0.2 ml | Subcutaneous | 4 mice |
| T3 | LAP/QAC† | 4 | 0,14 | 0.2 ml | Subcutaneous | 4 mice |
| T4 | Ovalbumin in LAP* | 4 | 0,14 | 0.2 ml | Subcutaneous | 4 mice |
| T5 | Ovalbumin in LAP/QAC† | 4 | 0,14 | 0.2 ml | Subcutaneous | 4 mice |
| T6 | Ovalbumin | 4 | 0,14 | 0.2 ml | Subcutaneous | 4 mice |
| *Lecithin Acrylic Polymer †Lecithin Acrylic Polymer /Quil A Cholesterol | | | | | | |

**Table 2. Treatment Group Vaccine Components: $\mu$g per 0.2 ml Dose**

| Treatment Group | LAP* | QAC** | Ovalbumin |
|---|---|---|---|
| T1 | --- | --- | --- |
| T2 | 1000[1] | --- | --- |
| T3 | 1000 | 50[2] | --- |
| T4 | 1000 | --- | 50 |
| T5 | 1000 | 50 | 50 |
| T6 | --- | --- | 50 |
| *Lecithin Acrylic Polymer **Quil A Cholesterol [1]600 $\mu$g CP; 400 $\mu$g Carbopol 934P [2]25 $\mu$g Quil A; 25 $\mu$g Cholesterol | | | |

METHODS

Study animals were subjected to vaccines at days 0 and 14, and spleens were harvested 48 hours after the second vaccination (day 15) (Table 20). Concentrations of treatment group vaccines are shown in Table 21. Splenic

35

lymphocytes from each animal were isolated by gradient centrifugation and cultured in vitro with media, ConA (10 $\mu$m/ml), LAP (6.2 $\mu$g/ml), LAP/QAC (6.2/0.062 $\mu$g/ml), or OVA (10 $\mu$g/ml). Additional in vitro treatments (culture with LAP + OVA and LAP/QAC + OVA) were performed if there were sufficient numbers of lymphocytes. For the additional treatments, OVA was added to cells first, and LAP or LAP/QAC was added to cells last. At approximately 24 and 48 hours in culture, samples were collected and RNA was isolated. RNA samples were analyzed by real time RT-PCR assays for expression of cytokine genes (TNF-$\alpha$, IL-4, IFN-$\gamma$). Levels of cytokine gene expression were normalized to expression of one housekeeping gene: hypoxanthine guanine phosphoribosyl transferase (HPRT) or acidic ribosomal phosphoprotein PO (ARBP). Relative levels of cytokine gene expression were calculated, using the normalized values, for in vitro treatments with respect to the media controls. A positive result is indicated by a relative expression ratio greater than 2, which shows cytokine gene expression for in vitro treated samples that is at least twice the level of expression observed in media control samples.

RESULTS

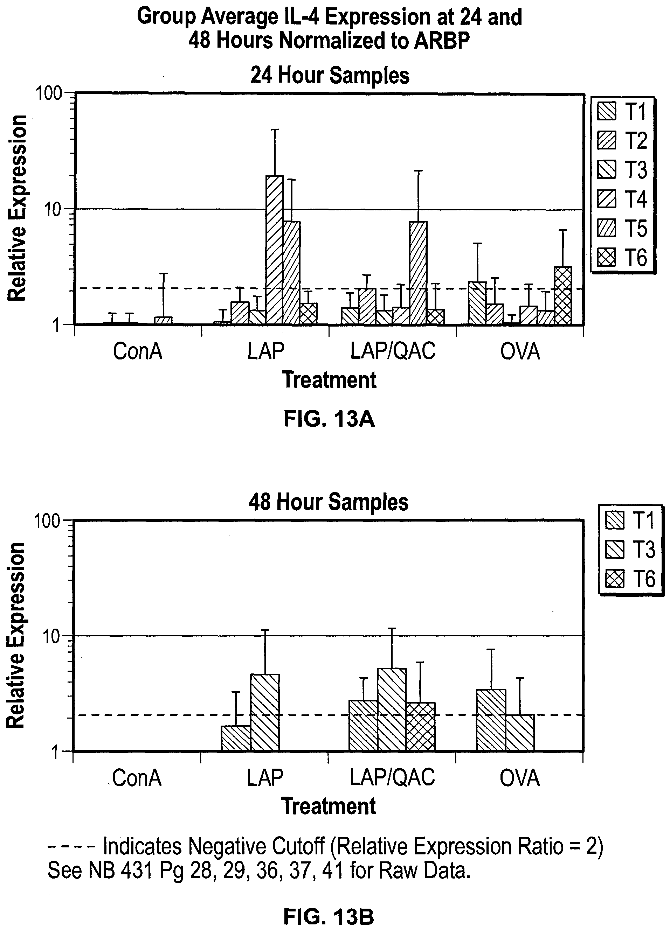

Relative expression of IL-4 mRNA was evaluated for samples from all treatment groups after 24 to 48 hours in culture (Figure 13A, B). Samples collected after 24 hours in culture with treatments as listed above (see Methods) using ARBP as the housekeeping gene for normalization purposes. The highest relative expression levels of IL-4 were observed in samples from vaccine treatment groups T4 (LAP + OVA) and T5 (LAP/QAC + OVA). Samples from vaccine treatment groups T1 (no vaccine), T3 (LAP/QAC), and T6 (OVA) were also evaluated at 48 hours in culture using ARBP as the housekeeping gene. Increased IL-4 expression was observed for some animals in each group after 48 hours in culture with LPA, LAP/QAC or OVA. Unexpectedly, ConA-treated samples did not have increased levels of IL-4 expression with respect to media controls; however, increases observed in samples from T4 and T5 indicate that cells were viable and were able to produce cytokines.

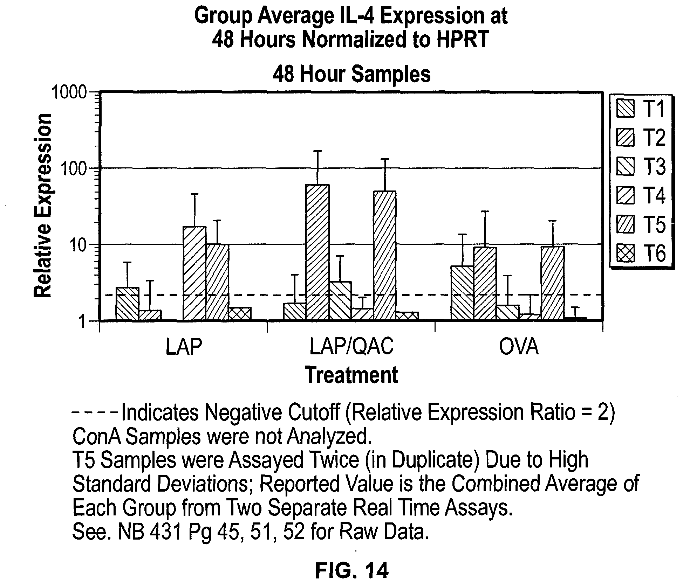

In order to determine whether relative expression ratios would be greater if an alternative housekeeping gene were used, samples collected after 48 hours in culture were assayed using HPRT as the housekeeping gene for normalization purposes (Figure 14). Increased relative expression of IL-4 was observed for vaccine treatment groups T4 (LAP + OVA) and T5 (LAP/QAC + OVA) after in vitro stimulation with LAP. In vitro treatment with LAP/QAC stimulated IL-4 mRNA production in samples from T2 (LAP) and T5 (LAP/QAC +OVA). Increases in relative IL-4 mRNA expression were also seen in cells from vaccine treatment groups T1 (no vaccine), T2 (LAP), and T5 (LAP/QAC + OVA) after incubation with OVA.

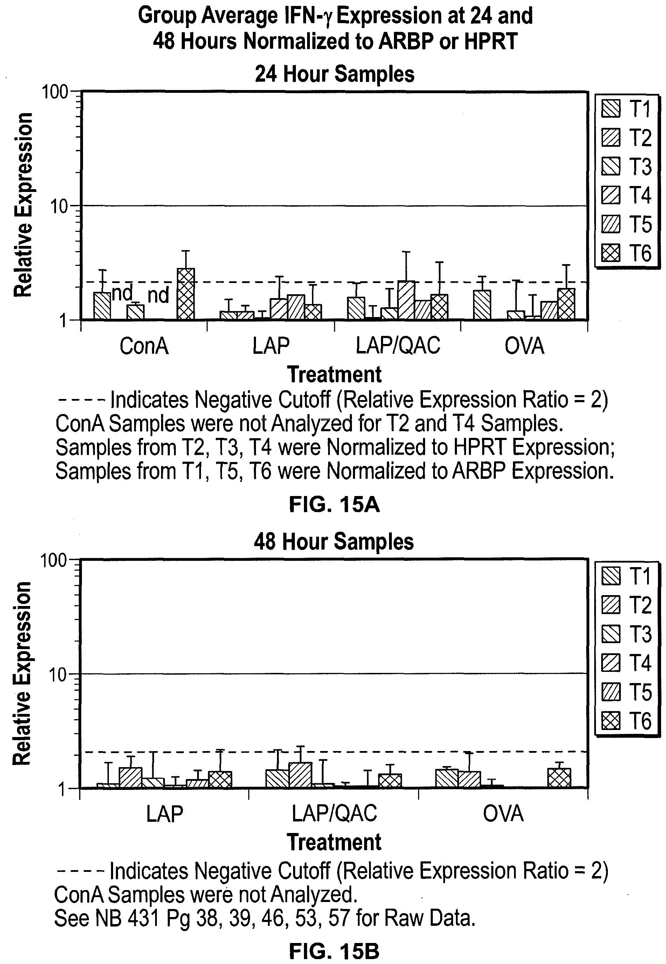

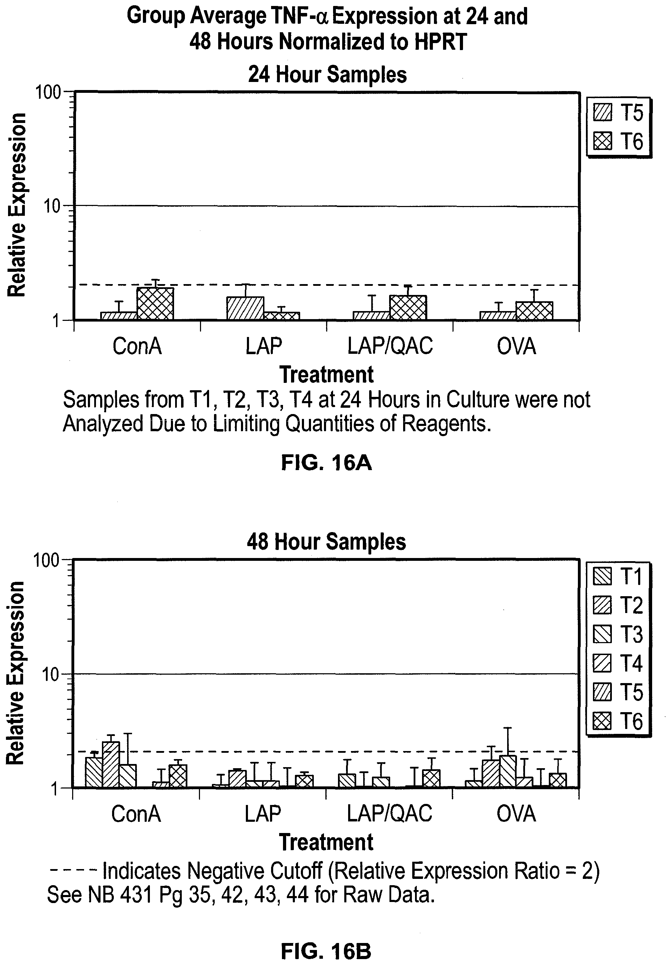

Relative expression levels of IFN-$\gamma$ and TNF-$\alpha$ were also evaluated for in vitro stimulated samples (Figure 15 A, B; Figure 16 A, B). No significant increases in these cytokines were observed. Positive control samples (ConA stimulated) were evaluated for selected samples; however, these control samples did not have increased levels of either cytokine in comparison to negative control samples (media treated).

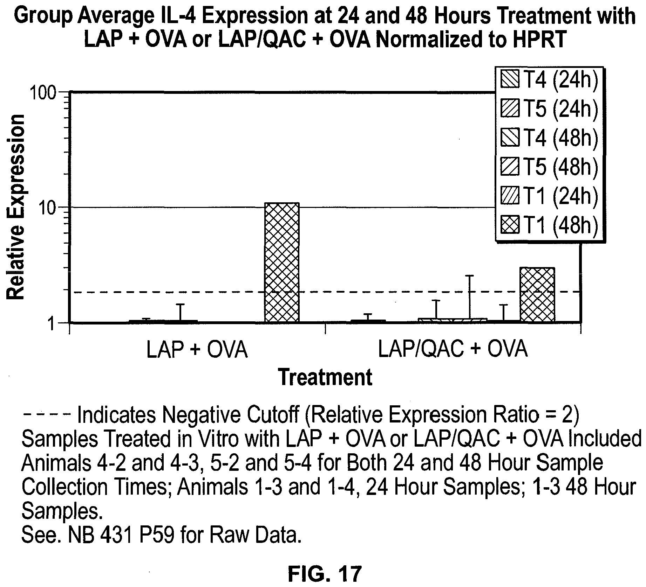

Samples from some animals were treated in vitro with LAP + OVA or LAP/QAC + OVA and relative expression of IL-4 mRNA was evaluated (Figure 17). Increases in IL-4 expression were observed for 48 hour samples from one animal (1-3) from treatment group T1 after in vitro stimulation with LAP + OVA and LAP/QAC + OVA.

Analysis of samples treated in vitro with LAP + OVA or LAP/QAC were also analyzed for relative expression of IFN$\gamma$ with HPRT as the housekeeping gene. Relative expression ratios for these samples were all less than 1, indicating that IFN$\gamma$ expression levels in treated cells were lower than the levels observed for media controls (data not shown).

CONCLUSION

The highest average relative expression ratios were observed for IL-4/ARBP in samples collected at 24 hours of culture. Samples from treatment groups T4 (LAP + OVA) and T5 (LAP/QAC + OVA) showed the greatest increases in relative expression of IL-4 message (Figure 1A, B). Analysis of samples from individual animals showed that there were large variations in IL-4 expression ratios among animals in these treatment groups. Cells from one animal in treatment group T1 had increased IL-4 mRNA expression after 48 hours in vitro stimulation with LAP + OVA and LAP/QAC + OVA.

Positive control samples (ConA stimulated) did not show increased cytokine expression as expected. Increases observed in IL-4 expression in some samples indicate that cells were capable of producing cytokines; however, further optimization of assay conditions is needed to determine culture times at which cytokine expression is at a maximum.

No significant increases in relative expression levels of IFN-$\gamma$ or TNF-$\alpha$ were observed. Because ConA positive controls did not show increased expression of IFN$\gamma$ or TNF-$\alpha$, further optimization of assays for detection of these cytokines may be warranted.

Having described the invention with reference to particular compositions, theories of effectiveness, and the like, it will be apparent to those of skill in the art that it is not intended that the invention be limited by such illustrative embodiments or mechanisms, and that modifications can be made without departing from the scope or spirit of the invention, as defined by the appended claims. It is intended that all such obvious modifications and variations be included within the scope of the present invention as defined in the appended claims. The claims are meant to cover the claimed components and steps in any sequence which is effective to meet the objectives there intended, unless the context specifically indicates to the contrary.

The following clauses set out further aspects of the present invention.

1. An adjuvant composition comprising a lecithin, a polymer, a glycoside and a sterol wherein the lecithin to polymer ratio is 1: 10 to 10: 1 by weight and wherein the weight of the glycoside and sterol combined is less than 1% of the weight of the lecithin and polymer combined.

2. The adjuvant composition of clause 1 wherein the glycoside is Quil A and the sterol is cholesterol.

3. The adjuvant composition of clause 2 wherein the polymer is an acrylic polymer selected from the group consisting of polyacrylic acid, methacrylic acid, methacrylate, acrylamide, acrylate, poly (acrylamide-co butyl, methacrylate), acrylic-methacrylic acid, acrylic-acrylamide, poly (methacrylate) and alkyl-esters of poly acrylic acid.

4. The adjuvant composition of clause 2 further comprising an antigen.

5. The adjuvant composition of clause 2 wherein the antigen is DNA based.

6. The adjuvant composition of clause 5 further comprising calcium phosphate.

7. An adjuvant composition consisting of a lecithin, a polymer, a glycoside and a sterol.

8. The adjuvant composition of clause 7 wherein the polymer is an acrylic polymer, the glycoside is Quil A and the sterol is cholesterol.

9. The adjuvant composition of clause 7 further consisting of a DNA based antigen wherein the adjuvant composition and DNA based antigen provide a vaccine.

10. The adjuvant composition of clause 9 further consisting of calcium phosphate.

11. A method for preparing a vaccine comprising:

combining a dry lecithin, an acrylic polymer, a glycoside and a sterol, wherein the dry lecithin to acrylic polymer are at a ratio of 1:10 to 10:1 by weight;
suspending the combined lecithin, acrylic polymer, glycoside and sterol in saline or water; and
mixing the lecithin, acrylic polymer, glycoside and sterol sufficiently to form a netlike structure;
wherein an antigen is added before or after suspension of the dry lecithin, acrylic polymer, glycoside and sterol such that a vaccine is formed and wherein the vaccine is used to elicit an immune response against an infectious agent, cancer, hormone, or autoimmune disease.

12. The method of clause 11 wherein the antigen is DNA based and further comprising addition of calcium phosphate prior to or after mixing of the lecithin, acrylic polymer, glycoside and sterol.

13. The method of clause 11 wherein the glycoside is Quil A and the sterol is cholesterol.

14. The method of clause 11 wherein the combined weight of glycoside and sterol is less than 1% of the weight of the lecithin and acrylic polymer.

## Claims

1. An adjuvant composition comprising a lecithin, a polymer, Quil A and cholesterol wherein the lecithin to polymer ratio is 1: 10 to 10: 1 by weight and wherein the weight of the Quil A and cholesterol combined is up to about 10% of the weight of the lecithin and polymer combined.

2. The adjuvant composition of claim 1 wherein the polymer is an acrylic polymer selected from the group consisting of polyacrylic acid, methacrylic acid, methacrylate, acrylamide, acrylate, poly (acrylamide-co butyl, methacrylate), acrylic-methacrylic acid, acrylic-acrylamide, poly (methacrylate) and alkyl-esters of poly acrylic acid.

3. The adjuvant composition of claim 1 further comprising an antigen.

4. The adjuvant composition of claim 1 wherein the antigen is DNA based.

5. The adjuvant composition of claim 4 further comprising calcium phosphate.

6. The adjuvant composition according to claim 1 wherein the composition consists of a lecithin, a polymer, Quil A and cholesterol, wherein the lecithin to polymer ratio is 1: 10 to 10: 1 by weight and wherein the weight of the Quil A and cholesterol combined is up to about 10% of the weight of the lecithin and polymer combined.

7. The adjuvant composition of claim 6 wherein the polymer is an acrylic polymer.

8. A vaccine composition consisting of a lecithin, a polymer, a glycoside and a sterol, wherein the lecithin to polymer ratio is 1: 10 to 10: 1 by weight and wherein the weight of the glycoside and sterol combined is up to about 10% of the weight of the lecithin and polymer combined.

9. The vaccine composition of claim 8 further consisting of calcium phosphate.

10. A method for preparing a vaccine comprising:

combining a dry lecithin, an acrylic polymer, a glycoside and a sterol, wherein the dry lecithin to acrylic polymer are at a ratio of 1:10 to 10:1 by weight, and wherein the weight of the glycoside and sterol combined is up to about 10% of the weight of the lecithin and polymer combined;
suspending the combined lecithin, acrylic polymer, glycoside and sterol in saline or water; and
mixing the lecithin, acrylic polymer, glycoside and sterol sufficiently to form a netlike structure;
wherein an antigen is added before or after suspension of the dry lecithin, acrylic polymer, glycoside and sterol such that a vaccine is formed and wherein the vaccine is used to elicit an immune response against an infectious agent, cancer, hormone, or autoimmune disease; and

wherein the glycoside is Quil A and the sterol is cholesterol.

11. The method of claim 10 wherein the antigen is DNA based and further comprising addition of calcium phosphate prior to or after mixing of the lecithin, acrylic polymer, glycoside and sterol.

FIG. 1

**FIG. 2A**

FIG. 2B

Seroconversion Rates and Avian Influenza Hemagglutination Inhibition (HAI) Geometric Mean Titer Results

| Trt. No. | Treatment Group | | Number of Birds Seroconverting / Total Birds | | Avian Influenza Geometric Mean Titers* | |
|---|---|---|---|---|---|---|
| | Antigen Adjuvant | Processing Method | Day 14 | Day 28 | Day 14 | Day 28 |
| T1 | CHO lysate** NT-1 Extract# LAP/QAC## | Mixing | 0 / 6 | 0 / 6 | 4.0 | 4.0 |
| T2 | CHO-HA-01-02† LAP/QAC## | Mixing | 2 / 6 | 5 / 5 | 8.0 | 48.5 |
| T3 | CHO-HA-01-02† LAP/QAC## | Microfluidize Non-clarified | 5 / 6 | 5 / 6 | 11.3 | 22.6 |
| T4 | CHO-HA-01-02† LAP/QAC## | Microfluidize Clarified | 3 / 6 | 5 / 6 | 9.0 | 28.5 |
| T5 | CHO-HA-01-02† LAP/QAC## | Silverson Non-clarified | 4 / 6 | 4 / 6 | 14.3 | 22.6 |
| T6 | CHO-HA-01-02† NT-1 Extract# LAP/QAC## | Mixing | 4 / 6 | 5 / 6 | 10.1 | 32.0 |
| T7 | CHO-HA-01-02† NT-1 Extract# LAP/QAC## | Microfluidize Non-clarified | 3 / 6 | 5 / 6 | 10.1 | 18.0 |
| T8 | CHO-HA-01-02† NT-1 Extract# LAP/QAC## | Microfluidize Clarified | 3 / 6 | 6 / 6 | 9.0 | 20.2 |
| T9 | CHO-HA-01-02† NT-1 Extract# LAP/QAC## | Silverson Non-clarified | 2 / 6 | 5 / 6 | 5.7 | 25.4 |
| T10 | H5N9 AIV†† LAP/QAC## | Mixing | 3 / 6 | 6 / 6 | 8.0 | 228.1 |

*Avian Influenza Geometric Mean Titers - Titers of < 8 were Factored as 4 for Calculation of Geometric Mean Titers.
**CHO lysate - Non-transfected CHO Cells.
#NT-1 Extract - Nicotiana Tabacum, lyophilized, non-transformed Clarified Cell lysate.
##LAP/QAC - Lecithin Acrylic Copolymer Plus Quil A Cholesterol.
†CHO-HA-01-02 - CHO Cells Transfected with H5 Hemagglutinin.
††H5N9 AIV - Inactivated H5N9 Avian Influenza Virus A/Turkey/Wisconsin/68.

FIG. 3

| Effect of Adjuvants on Vaccine Potency | | | | | | | | | | |
|---|---|---|---|---|---|---|---|---|---|---|
| Experimental Design: 2nd Generation Ad5, SC, 2 Sites, Various Doses +/- Adjuvants, Challenge 21 dpv (10-fold Higher than OIE Recommendation) | | | | | | | | | | |
| Vaccine | Dose (PFU) | Adjuvant | Animal # | Viremia | Shedding Virus | Clinical Score | SN | 3ABC ELISA | RT-PCR | 3D (RIP) |
| Ad5-Blue | $2 \times 10^6$ | – | 29 | $2/1.0 \times 10^5/2$ | $2/8.2 \times 10^1/2$ | 2/16 | 0.1/3 | SP | SP | SP |
| | | | 30 | $2/4.0 \times 10^5/3$ | $2/5.6 \times 10^2/2$ | 2/17 | 0.1/3 | SP | SP | SP |
| | | | 31 | $2/2.7 \times 10^5/2$ | $2/8.2 \times 10^2/2$ | 2/17 | 0.1/3.3 | SP | SP | SP |
| Ad5-CI-A24-2B | $2 \times 10^6$ | – | 12 | 0 | 0 | 0 | 1.2/2.4 | N | N | N |
| | | | 13 | 0 | 0 | 0 | 1.2/2.1 | N | N | N |
| | | | 14 | 0 | 0 | 0 | 1.2/2.1 | N | N | N |
| Ad5-CI-A24-2B | $4 \times 10^7$ | – | 26 | 0 | 0 | 3/3 | 1.2/3.6 | SP | WP | SP |
| | | | 27 | 0 | 0 | 3/2 | 1.2/4.2 | SP | N | P |
| | | | 28 | 0 | 0 | 6/1 | 0.9/3.6 | WP | WP | WP |
| Ad5-CI-A24-2B | $4 \times 10^7$ | Adj E 1:5 | 15 | 0 | 0 | 0 | 0.9/2.1 | N | N | N |
| | | | 17 | 0 | 0 | 0 | 1.2/2.4 | N | N | N |
| | | | 18 | 0 | 0 | 0 | 1.8/2.4 | N | N | N |
| Ad5-CI-A24-2B | $4 \times 10^7$ | pICLC 1mg | 23 | 0 | 0 | 0 | 1.5/3 | N | N | N |
| | | | 24 | 0 | 0 | 6/1 | 1.2/3.9 | SP | N | WP |
| | | | 25 | 0 | 0 | 6/1 | 1.2/3.3 | SP | N | WP |
| Adjuvant E Enhances Potency At Least 5-fold | | | | | | | | | | |

**FIG. 4**

## Percent Reduction of Alamar
## Blue 42 Hrs PT (Rx 1)

Percent Reduction of Alamar Blue, 42 Hours
Post Treatment (PT)

Cell Density = $1 \times 10^6$/ml

**FIG. 5**

# Percent Reduction of Alamar Blue 50 Hrs PT (Rx 1)

Percent Reduction of Alamar Blue, 50 Hours Post Treatment (PT)

Cell Density = 1 X $10^6$/ml

## FIG. 6

## Percent Reduction of Alamar Blue 68 Hrs PI (Rx 1)

Percent Reduction of Alamar Blue, 68 Hours
Post Treatment (PT)

Cell Density = 1 X 10$^6$/ml

**FIG. 7**

## Percent Reduction of AB 42 Hrs PT (Rx 2)

Percent Reduction of Alamar Blue, 42 Hours
Post Treatment (PT)

Cell Density = 10 X $10^6$/ml

**FIG. 8**

## Percent Reduction of AB 50 Hrs PT (Rx 2)

Percent Reduction of Alamar Blue, 50 Hours
Post Treatment (PT)

Cell Density = 10 X 10$^6$/ml

**FIG. 9**

**Percent Reduction of AB
68 Hrs P. I., (Rx 2)**

Percent Reduction of Alamar Blue, 50 Hours
Post Treatment (PT)

Cell Density = 10 X $10^6$/ml

**FIG. 10**

**FIG. 11**

FIG. 12

## Group Average IL-4 Expression at 24 and 48 Hours Normalized to ARBP

### 24 Hour Samples

**FIG. 13A**

### 48 Hour Samples

- - - - Indicates Negative Cutoff (Relative Expression Ratio = 2)
See NB 431 Pg 28, 29, 36, 37, 41 for Raw Data.

**FIG. 13B**

**Group Average IL-4 Expression at
48 Hours Normalized to HPRT**

- - - - Indicates Negative Cutoff (Relative Expression Ratio = 2)
ConA Samples were not Analyzed.
T5 Samples were Assayed Twice (in Duplicate) Due to High
Standard Deviations; Reported Value is the Combined Average of
Each Group from Two Separate Real Time Assays.
See. NB 431 Pg 45, 51, 52 for Raw Data.

**FIG. 14**

## Group Average IFN-γ Expression at 24 and 48 Hours Normalized to ARBP or HPRT

### 24 Hour Samples

---- Indicates Negative Cutoff (Relative Expression Ratio = 2)
ConA Samples were not Analyzed for T2 and T4 Samples.
Samples from T2, T3, T4 were Normalized to HPRT Expression;
Samples from T1, T5, T6 were Normalized to ARBP Expression.

**FIG. 15A**

### 48 Hour Samples

---- Indicates Negative Cutoff (Relative Expression Ratio = 2)
ConA Samples were not Analyzed.
See NB 431 Pg 38, 39, 46, 53, 57 for Raw Data.

**FIG. 15B**

## Group Average TNF-α Expression at 24 and 48 Hours Normalized to HPRT

### 24 Hour Samples

Samples from T1, T2, T3, T4 at 24 Hours in Culture were not Analyzed Due to Limiting Quantities of Reagents.

**FIG. 16A**

### 48 Hour Samples

- - - - Indicates Negative Cutoff (Relative Expression Ratio = 2) See NB 431 Pg 35, 42, 43, 44 for Raw Data.

**FIG. 16B**

**Group Average IL-4 Expression at 24 and 48 Hours Treatment with LAP + OVA or LAP/QAC + OVA Normalized to HPRT**

- - - - Indicates Negative Cutoff (Relative Expression Ratio = 2)
Samples Treated in Vitro with LAP + OVA or LAP/QAC + OVA Included
Animals 4-2 and 4-3, 5-2 and 5-4 for Both 24 and 48 Hour Sample
Collection Times; Animals 1-3 and 1-4, 24 Hour Samples; 1-3 48 Hour
Samples.
See. NB 431 P59 for Raw Data.

**FIG. 17**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 61609783
- US 4917892 A, Speaker
- US 5132117 A, Speaker
- US 4740365 A, Yukimatsu
- US 5451411 A, Gombotz
- US 5352448 A, Bowersock
- US 5674495 A, Bowersock
- US 4944942 A, Brown
- US 5500161 A, Andrianov
- US 6015576 A, See

- US 5811128 A, Tice
- US 5565209 A, Rijke
- US 5084269 A, Kullenberg
- US 5026543 A, Rijke
- US 5567433 A, Collins
- US 5091188 A, Haynes
- US 3929678 A, Laughlin
- US 125577
- US 5716637 A, Anselem

**Non-patent literature cited in the description**

- **SABIN, A. B.** Vaccination at the portal of entry of infectious agents. *Dev Biol Stand,* 1976, vol. 33, 3-9
- **O'HAGEN, D.** Oral Delivery of Vaccines: Formulation and Clinical Pharmacokinetic Considerations. *Clin. Pharmacokinet.,* 1992, vol. 22 (1), 1-10
- **MESTECKY, JI.** The Common Mucosal Immune System and Current Strategies for Induction of Immune Responses in External Secretions. *J Clin Immunol.,* vol. 7 (4), 265-76

- **PERIWAL, SB.** Orally administered microencapsulated reovirus can bypass suckled, neutralizing maternal antibody that inhibits active immunization of neonates. *J Virol,* April 1997, vol. 71 (4), 2844-50
- **SWENSON, ES ; WJ CURATOLO.** Means to Enhance Penetration (2) Intestinal permeability enhancement for proteins, peptides and other polar drugs: mechanisms and potential toxicity. *Advanced Drug Delivery Reviews,* 1992, vol. 8, 39-92